# EUROPÄISCHE PATENTSCHRIFT

(11) **EP 2 990 036 B1**
(45) Veröffentlichungstag und Bekanntmachung des Hinweises auf die Patenterteilung: **10.04.2019**
(21) Anmeldenummer: 14179087.3
(22) Anmeldetag: 30.07.2014
(51) Int. Cl.: A61K 31/352, A61P 1/14, A23L 33/10

(54) **Hydroxyflavone als Appetitanreger**
Hydroxyflavones for stimulating appetite
Hydroxyflavones pour stimulation d'appétit

(43) Veröffentlichungstag der Anmeldung: 02.03.2016
(73) Patentinhaber: Symrise AG, 37603 Holzminden (DE)
(72) Erfinder: Somoza, Veronika, 3400 Weidling (AT); Rohm, Barbara, 1230 Wien (AT); Liszt, Kathrin, 1220 Wien (AT); Pignitter, Marc, 1160 Wien (AT); Hochkogler, Christina, 2230 Gänserndorf (AT); Ley, Jakob, 37603 Holzminden (DE); Widder, Sabine, 37603 Holzminden (DE); Krammer, Gerhard, 37603 Holzminden (DE)
(74) Vertreter: Eisenführ Speiser

(56) Entgegenhaltungen:
- EP-A1- 2 614 727
- EP-A1- 2 767 174
- US-A- 5 621 011
- DATABASE WPI Week 201323 28. Januar 2013 (2013-01-28) Thomson Scientific, London, GB; AN 2013-B86645 XP002734505, & KR 2013 0010192 A (KOREA FOOD RES INST) 28. Januar 2013 (2013-01-28)
- DATABASE WPI Week 201224 22. März 2012 (2012-03-22) Thomson Scientific, London, GB; AN 2012-D41954 XP002734506, & WO 2012/036344 A1 (KOREA FOOD RES INST) 22. März 2012 (2012-03-22)
- DATABASE WPI Week 200901 10. Juli 2008 (2008-07-10) Thomson Scientific, London, GB; AN 2009-A16368 XP002734507, & JP 2008 156297 A (TSUMURA & CO) 10. Juli 2008 (2008-07-10)
- KIM H Y ET AL: "Hesperetin stimulates cholecystokinin secretion in enteroendocrine STC-1 cells", BIOMOLECULES AND THERAPEUTICS 2013 KOREAN SOCIETY OF APPLIED PHARMACOLOGY KOR, Bd. 21, Nr. 2, 2013, Seiten 121-125, XP002734508, ISSN: 1976-9148

## Beschreibung

### GEBIET DER ERFINDUNG

Die Erfindung betrifft ein Medikament enthaltend Hydroxyflavanone der Formel (I) oder deren Salze zur Verwendung bei der Behandlung von Appetitlosigkeit, die nicht-therapeutische Verwendung von Hydroxyflavonen der Formel (I) oder deren Salze, als Appetitanreger, vorzugsweise in oral konsumierbaren Produkten, welche ausgewählt sind aus der Gruppe bestehend aus Lebensmitteln (insbesondere flüssige oder feste, einschließlich Halbfertigwaren), Futtermitteln und Arzneimitteln (pharmazeutische Zubereitungen).

### STAND DER TECHNIK

Oft verspüren ältere Personen einen geringeren Appetit auf Nahrung, was in extremen Fällen sogar zu einer Mangelernährung führen kann. Verringerter Appetit kann verschiedene Ursachen habe, so z.B. allgemein die verringerte sensorische Empfindlichkeit, Erschöpfung, Allgemeinerkrankungen, Medikamenteneinnahme oder auch unangenehme Erfahrungen im Zusammenhang mit Mundtrockenheit und Nahrungsaufnahme. Um den Appetit auf die Nahrung zu erhöhen, kann im Allgemeinen auf verbesserte Rezepturen z.B. bezüglich Farbe, Aroma, Geschmack und auch Textur zurückgegriffen werden. Allerdings ist es auch denkbar, durch Beeinflussung von bestimmten Hormonen oder Neurotransmittern das Gefühl des Appetits zu steigern.

Um eine erhöhten Aufnahme kalorisch relevanter Nahrungsmittelbestandteile zu bewirken, ist es ein Wunsch als sicher bewertete, bereits zugelassene und allgemein akzeptierte appetitsteigernde Substanzen zu finden, die das Hungergefühl und den natürlichen Appetit verstärken können. Es ist bekannt, dass durch Erniedrigung der Serotonin-Ausschüttung in bestimmten Hirnarealen bei gleichzeitiger Exposition mit Nährstoffen durch die aufgenommenen oral konsumierbaren Produkte (insbesondere Lebensmittel) der Appetit gesteigert werden kann. Eine Steigerung des Hungergefühls wird, unter anderem, durch verstärkte Ausschüttung des gastrointestinalen Hungerhormons Ghrelin erzielt, welches durch eine hohe Konzentration freier Fettsäure im Blut gehemmt wird (Gormsen et al. 2006; Eur J Endocrinology). Eine Erniedrigung freier Fettsäuren im Blut führt somit zu einer Steigerung des Appetits und kann durch eine verstärkte Aufnahme von freien Fettsäuren durch periphere Zellen (beispielsweise Adipozyten) hervorgerufen werden.

Die Aufgabe der vorliegenden Erfindung hat somit darin bestanden, Substanzen zu identifizieren, die insbesondere den Seretoninspiegel und / oder die Konzentration freier Fettsäuren im Blut beeinflussen und reduzieren bzw. senken können, um den Appetit anzuregen. Es war Bestandteil der vorliegenden Aufgabe appetitanregende Substanzen zu identifizieren, die insbesondere lebensmittelrechtlich konform sind und somit toxikologisch unbedenklich einsetzbar sind. Ebenfalls war es Aufgabe der vorliegenden Erfindung appetitanregende Substanzen zu identifizieren, die einfach zugänglich sind und möglichst natürlich vorkommen, bevorzugt als pflanzlicher Extrakt gewonnen werden kann.

### BESCHREIBUNG DER ERFINDUNG

Gegenstand der Erfindung betrifft daher bestimmte Verwendungen bzw. Anwendungen von ausgewählten Verbindungen der Formel (I) 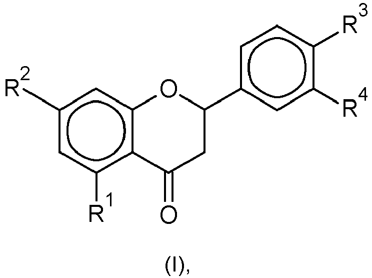 oder deren Salze, wobei
R¹ H oder OH,
R² H, OH, oder OCH₃,
R³ H, OH, oder OCH₃,
   und
R⁴ H, OH, oder OCH₃
darstellt, wobei mindestens zwei der Reste R¹, R², R³ oder R⁴ jeweils ein OH darstellen, zur Behandlung von Appetitlosigkeit, insbesondere von
Verbindungen der Formel (I) wobei
R¹ OH,
R² OH, oder OCH₃,
R³ OH, oder OCH₃,
   und
R⁴ OH, oder OCH₃
darstellt, wobei mindestens drei der Reste R¹, R², R³ oder R⁴ jeweils ein OH darstellen.

Erfindungsgemäß sind die Verbindungen ausgewählt aus der Gruppe bestehend aus Homoeriodictyol (1), Eriodictyol (2), und/oder Sterubin (4).

Dementsprechend betrifft ein erster Aspekt der vorliegenden Erfindung nicht-therapeutische Verwendungen von Homoeriodictyol (1), Eriodictyol (2), und/oder Sterubin (4) in der Ernährung oder dem Genuss dienenden oral zu konsumierenden Lebensmitteln zur Beeinflussung des Serotoninspiegels und der Konzentration freier Fettsäuren im Blut zur Steigerung des Appetits.

Zudem betrifft der Gegenstand der Erfindung die nicht-therapeutische Verwendung eines der Ernährung oder dem Genuss dienenden Lebensmittels, vorzugsweise eines Nahrungsergänzungsmittels, umfassend
(i) mindestens eine Verbindungen der Formel (I) oder deren Salze, wobei die Verbindungen der Formel (I) ausgewählt sind aus der Gruppe bestehend aus: Homoeriodictyol (1), Eriodictyol (2) und Sterubin (4),
(ii) mindestens einen festen Trägerstoff, und
(iii) einen oder mehrere Aromastoffe,
zur Beeinflussung des Serotoninspiegels und der Konzentration freier Fettsäuren im Blut zur Steigerung des Appetits.

Der Gegenstand der Erfindung betrifft des Weiteren ein Medikament, enthaltend Verbindungen der Formel (I) oder deren Salze, wobei die Verbindungen der Formel (I) ausgewählt sind aus der Gruppe bestehend aus Homoeriodictyol (1), Eriodictyol (2) und Sterubin (4), zur Verwendung bei der Behandlung von Appetitlosigkeit.

In einer bevorzugten Ausgestaltung umfasst ein erfindungsgemäßes Medikament
(i) mindestens eine Verbindung nach Anspruch 1 oder deren Salze
(ii) mindestens einen festen Trägerstoff,
(iii) einen oder mehrere Aromastoffe,
mit der Maßgabe, dass sich die Komponenten (i), (ii) und (iii) mit gegebenenfalls weiteren Inhaltstoffen zu 100 Gew.-% addieren, bezogen auf die Gesamtmenge der gesamten Stoffmischung.

In einer weiteren bevorzugten Ausgestaltung ist ein erfindungsgemäßes Medikament dadurch gekennzeichnet, dass im Salz die Gegenkationen ausgewählt sind aus der Gruppe bestehend aus: Ammoniumionen, Trialkylammoniumionen, Natrium-, Kali-um-, Magnesium-, oder Calciumkationen.

Bevorzugt hat das Stereozentrum der Verbindungen der Formel (I) an C-2 jeweils (R)- oder (S)-Konfiguration.

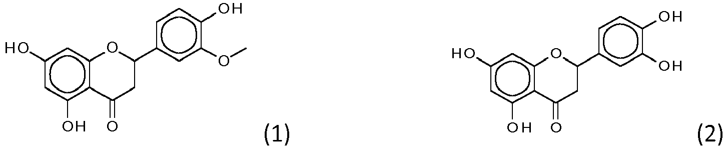

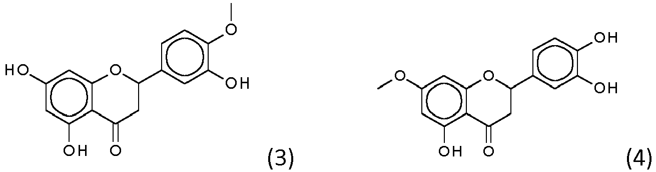

Vorzugsweise werden Mischungen beider Konfigurationen (R)- und (S), besonders bevorzugt einer an (S)-Konfiguration angereicherten Mischung eingesetzt. Vorzugsweise werden Verbindungen der Formel (I) als (2R)- und /oder (2S)-Enantiomere eingesetzt, so dass eine bevorzugte Ausführungsform die Verwendung der Verbindungen der Formel (I) als (2R)- und /oder (2S)-Enantiomere ist.

Vorzugsweise werden Verbindungen der Formel (I) in Form Ihrer Salze verwendet, wobei die Gegenkationen in einer bevorzugten Ausführungsform ausgewählt sind aus der Gruppe bestehend aus: Ammoniumionen, Trialkylammoniumionen, Natrium-, Kalium-, Magnesium-, oder Calciumkationen. Hierbei ist das Mononatriumsalz der Verbindung (1), das Homoeriodictyolnatriumsalz (= HEDNa) besonders bevorzugt.

Natürliche Quellen und Zugänge zu den genannten Hydroxyflavanonen der Formel (I), insbesondere den Verbindungen (1) bis (4), sind im Stand der Technik bekannt. So werden z.B. in EP 1,258,200-B1, EP 1,998,636-B1 oder EP 2,017,272 B1 die Darstellung der genannten Hydroxyflavonen beschrieben. Bevorzugt werden hierbei Extrakte oder Isolate aus Herba Santa, wobei aufgereinigtes Homoeriodictyolnatriumsalz nach EP 1,258,200-B1 oder Homoeriodictyol-reiche Fraktionen wie in EP 2,633,885 beschrieben, ganz besonders bevorzugt zu Gewinnung eingesetzt werden. Insbesondere für Nahrungs- und Genussmittel zur Extraktion geeignete Lösungsmittel sind hierbei Wasser, Ethanol, Methanol, 1,2-Propylenglycol, Glycerin, Aceton, Dichlormethan, Essigsäureethylester, Diethylether, Hexan, Heptan, Triacetin, pflanzliche Öle oder Fette, superkritisches Kohlendioxid und deren Gemische.

Hydroxyflavanone der Formel (I) sind bisher als geschmacksmodulierende Aromastoffe bekannt (EP 1,258,200-B1, EP 1,998,636-B1). Ihre Wirkung auf den Serotoninspiegel und auf die Konzentration von freien Fettsäuren im Blut, insbesondere bei Säugetieren, vorzugsweise beim Menschen ist bisher nicht bekannt. Ebenso ist die daraus resultierende mögliche Verwendung zur Steigerung des Appetits so bisher nicht bekannt.

Demgemäß ist eine bevorzugte Ausführungsform der vorliegenden Erfindung die Verwendung der Verbindungen der Formel (I), insbesondere den Verbindungen (1) bis (4) oder Gemische davon, vorzugsweise Gemische der (R)- und / oder (S)- Enantiomere zur Beeinflussung des Serotoninspiegels und der Konzentration freier Fettsäuren im Blut, bei Säugetieren, vorzugsweise beim Menschen.

Ebenso ist eine bevorzugte Ausführungsform der vorliegenden Erfindung die Verwendung der Verbindungen der Formel (I), insbesondere den Verbindungen (1) bis (4) oder Gemische davon, vorzugsweise Gemische der (R)- und / oder (S)- Enantiomere als Mittel zur Steigerung des Appetits.

Im Sinne der vorliegenden Erfindungen ist stets unter Verbindungen bzw. Hydroxyflavanonen der Formel (I), insbesondere Verbindungen (1) bis (4) oder Gemische davon, vorzugsweise Gemische der (R)- und / oder (S)- Enantiomere oder Salze, zu verstehen. Vorzugsweise handelt es sich um eine an S- Konfiguration angereicherte Mischung der Verbindungen der Formel (I), bevorzugt an Verbindungen (1) bis (4).

Die Verwendung der Hydroxyflavanone der Formel (I) zur Appetitanregung hat nichts mit deren bereits bekannten geschmacksverbessernden Eigenschaften, insbesondere deren bittermaskierende Wirkung, zu tun. Im Gegensatz zu der vorliegenden Erfindung ist insbesondere bei der Verwendung als geschmacksverbessernde Substanz, die Gegenwart von bitter schmeckenden Stoffen notwendig, wohingegen diese bei der vorliegenden Erfindung nicht essentiell anwesend sein müssen. Insbesondere war es überraschend, dass die Hydroxyflavanone der Formel (I) neben der bereits bekannten Wirkung, auch einen Einfluss auf die Serotoninfreisetzung aufweisen und diese sowohl in neuronalen Zellkulturen als auch in vivo-Experimenten im Blutplasma verringern können.

Überraschenderweise hat sich zudem gezeigt, dass Hydroxyflavanone der Formel (I), in der Lage sind, bereits
(a) in einer Konzentration von etwa 0,001 µM bis 10 µM (ca. 0,03 µg/kg bis ca. 3 mg/kg) die Serotonin-Freisetzung um bis zu 70 % gegen die jeweilige Kontrolle zu senken, und ferner
(b) in einer Konzentration von etwa 0,1 µM die Fettsäureaufnahme in Adipozyten um 8% gegen die Kontrolle zu steigern.

Die angewendeten Konzentrationen für die erfindungsgemäßen Verwendungszwecke der Hydroxyflavanone der Formel (I) sind im Vergleich zu typischen Einsatzkonzentrationen (wie in EP 1,258,200-B1, EP 1,998,636-B1 beschrieben) zu gering um einen ausgeprägten Geschmackseffekt zu erzielen.

In einer bevorzugten Ausführungsform werden die Verbindungen der Formel (I) oder deren Salze in oralen pharmazeutischen Zubereitungen einformuliert.

Vorzugsweise werden Hydroxyflavanone der Formel (I) zur erfindungsgemäßen Anwendung in einem therapeutischen Verfahren bzw. in einer erfindungsgemäßen nicht-therapeutischen Verwendung eingesetzt, wobei die Hydroxyflavanone der Formel (I) alleine oder als Gemisch Bestandteil eines oral konsumierbaren Produktes eingesetzt werden, wobei
- das Hydroxyflavanon oder die Hydroxyflavanone in einer Konzentration von 30 mg/kg oder weniger vorliegt, bezogen auf die Gesamtmasse des oral konsumierbaren Produktes, vorzugsweise in einer Konzentration von 10 mg/kg oder weniger, besonders bevorzugt in einer Konzentration von 3 mg/kg oder weniger.

Bevorzugt ist ein erfindungsgemäß oral konsumierbares Produkt hierbei ausgewählt aus der Gruppe bestehend aus Lebensmitteln (insbesondere flüssige oder feste, einschließlich Halbfertigwaren), Futtermitteln und Arzneimitteln (pharmazeutische Zubereitungen). Insbesondere sind die Hydroxyflavone der Formel (I) vorliegenden Erfindung dazu geeignet zur Verwendung in Viehfutter für die Tierernährung, ins besonderer zur Beeinflussung des Serotoninspiegels und / oder der Konzentration freier Fettsäuren im Blut und somit zur Steigerung des Appetits bei Tieren, speziell bei Säugetieren. Ein weiterer Gegenstand der Erfindung umfasst daher auch ein Medikament enthaltend Verbindungen der Formel (I) besonderer zur Beeinflussung des Serotoninspiegels und / oder der Konzentration freier Fettsäuren im Blut von Säugetieren.

Ein weiterer Gegenstand der vorliegenden Erfindung ist die (kosmetische) Anwendung von Verbindungen der Formel (I) oder deren Salze 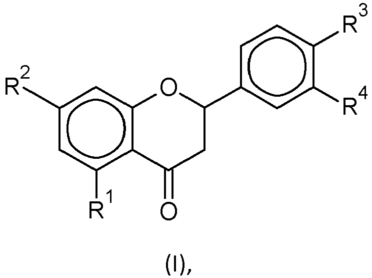 wobei
R¹ H oder OH,
R² H, OH, oder OCH₃,
R³ H, OH, oder OCH₃,
   und
R⁴ H, OH, oder OCH₃
darstellt, wobei mindestens zwei der Reste R¹, R², R³ oder R⁴ jeweils ein OH darstellen, in der Ernährung oder dem Genuss dienenden Lebensmitteln. Die kosmetische Anwendung richtet sich im speziellen auf die Wirkung von Hydroxyflavone der Formel (I) als appetitanregende Substanzen in kosmetisch gerichteten Erzeugnissen, die nicht pharmazeutische Mittel sind, d.h. nicht zu therapeutischen Zwecken eingesetzt werden.
Dabei sind die Verbindungen der Formel (I) ausgewählt aus der Gruppe bestehend aus: Homoeriodictyol (1), Eriodictyol (2) und Sterubin (4), oder Gemische davon, vorzugsweise Gemische der (R)- und / oder (S)- Enantiomere, besonders bevorzugt eine an S- Konfiguration angereicherte Mischung.

Ferner ist ein weiterer Aspekt der vorliegenden Erfindung die nicht-therapeutische Verwendung der Hydroxyflavone der Formel (I) zur Beeinflussung des Serotoninspiegels und / oder der Konzentration freier Fettsäuren im Blut zur Steigerung des Appetits. Vorzugsweise ist bei der genannten nicht-therapeutischen Anwendung bzw. Verwendung das der Ernährung oder dem Genuss dienenden Lebensmittel ein oral konsumierbares Produkt. Die nicht-therapeutische Verwendung kann eine kosmetische Verwendung bzw. Anwendung sein.
Ein weiterer Gegenstand der vorliegenden Erfindung ist die Verwendung von Hydroxyflavonen der Formel (I) oder deren Salze als Arzneimittel zur Verwendung bei der Behandlung von Appetitlosigkeit.

Im Rahmen des vorliegenden Textes umfasst der Begriff "Lebensmittel" eine Vielzahl von Produkten. Unter den Begriff Lebensmittel fallen insbesondere Produkte, wie sie weiter unten im Zusammenhang mit erfindungsgemäßen Lebensmitteln diskutiert werden.
Der Begriff "Lebensmittel" umfasst insbesondere Produkte, die gemäß der VERORDNUNG (EG) Nr. 178/2002 DES EUROPÄISCHEN PARLAMENTS UND DES RATES vom 28. Januar 2002 Lebensmittel sind. Gemäß dieser Verordnung sind "Lebensmittel" alle Stoffe oder Erzeugnisse, die dazu bestimmt sind oder von denen nach vernünftigem Ermessen erwartet werden kann, dass sie in verarbeitetem, teilweise verarbeitetem oder unverarbeitetem Zustand von Menschen aufgenommen werden. Zu "Lebensmitteln" zählen auch Getränke, Kaugummi sowie alle Stoffe - einschließlich Wasser-, die dem Lebensmittel bei seiner Herstellung oder Ver- oder Bearbeitung absichtlich zugesetzt werden.

Der Begriff "Futtermittel" umfasst im Rahmen des vorliegenden Textes alle Formen von Tiernahrung. Eine Vielzahl der nachstehend genannten Lebensmittel kann auch als Futtermittel eingesetzt werden.

Der Begriff "Arzneimittel" umfasst im Rahmen des vorliegenden Textes Stoffe oder Stoffzusammensetzungen, die als Mittel mit Eigenschaften zur Heilung oder zur Verhütung menschlicher oder tierischer Krankheiten bestimmt sind oder aber im oder am menschlichen oder tierischen Körper verwendet oder einem Menschen bzw. Tier verabreicht werden können, um entweder die menschlichen bzw. tierischen physiologischen Funktionen durch eine pharmakologische, immunologische oder metabolische Wirkung wiederherzustellen, zu korrigieren oder zu beeinflussen oder eine medizinische Diagnose zu erstellen. Arzneimittel können im Einzelfall zu nicht-therapeutischen, insbesondere kosmetischen Zwecken eingesetzt werden.

Es versteht sich, dass Lebensmittel oder Futtermittel durch den Zusatz von Stoffen oder Stoffzusammensetzungen, die als Mittel mit Eigenschaften zur Heilung oder zur Verhütung menschlicher oder tierischer Krankheiten bestimmt sind, in entsprechende Arzneimittel überführt werden können.

Um wegen des gesteigerten Appetits und der daher einhergehenden höheren Aufnahmemenge einen unerwünschten Anstieg des Körpergewichtes beim Konsumenten zu vermeiden, hat es sich als sinnvoll erwiesen, die potentielle kalorische Aufnahme zu begrenzen und erfindungsgemäße oral konsumierbare Produkte (insbesondere Lebensmittel, Futtermittel bzw. Arzneimittel) zum Verzehr anzubieten, die ihrerseits bereits eine geringe Energiedichte aufweisen. Demgemäß enthält ein bevorzugtes erfindungsgemäßes oral konsumierbares Produkt (insbesondere Lebensmittel, Futtermittel und Arzneimittel) nicht mehr als 200 kcal/100g des oral konsumierbaren Produktes, bevorzugt nicht mehr als 100 kcal/100g, besonders bevorzugt nicht mehr als 40 kcal/100g.

Bevorzugte erfindungsgemäße oral konsumierbare Produkte (insbesondere Lebensmittel, Futtermittel oder Arzneimittel) sind jegliche zum Verzehr geeignete der Ernährung, der Mundpflege oder dem Genuss dienende Zubereitungen oder Zusammensetzungen und sind regelmäßig Produkte, die dazu bestimmt sind, in die menschliche bzw. tierische Mundhöhle eingebracht zu werden, dort eine bestimmte Zeit zu verbleiben und anschließend entweder verzehrt (z.B. verzehrfertige Lebensmittel oder Futtermittel, siehe auch weiter unten) oder wieder aus der Mundhöhle entfernt zu werden (z.B. Kaugummis oder Mundpflegeprodukte oder medizinische Mundspülungen). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen oder Tier aufgenommen zu werden. Hierzu zählen auch Stoffe, die oral konsumierbaren Produkten (insbesondere Lebensmittel, Futtermittel und Arzneimittel) bei ihrer Herstellung, Verarbeitung oder Bearbeitung zugesetzt werden und dazu vorgesehen sind, in die menschliche oder tierische Mundhöhle eingebracht zu werden.

Zu den erfindungsgemäßen oral konsumierbaren Produkten (insbesondere Lebensmittel, Futtermittel und Arzneimittel) zählen auch Stoffe, die dazu bestimmt sind, in unverändertem, zubereitetem oder verarbeitetem Zustand vom Menschen oder Tier geschluckt und dann verdaut zu werden; zu den erfindungsgemäßen oral konsumierbaren Produkten gehören insoweit auch Umhüllungen, Überzüge oder sonstige Umschließungen, die dazu bestimmt sind, mit verschluckt zu werden, oder bei denen ein Verschlucken vorauszusehen ist. Der Begriff "oral konsumierbares Produkt" umfasst verzehrfertige Lebensmittel und Futtermittel, d.h. Lebensmittel oder Futtermittel, die hinsichtlich der für den Geschmack maßgeblichen Substanzen bereits vollständig zusammengesetzt sind. Unter den Begriffen "verzehrfertiges Lebensmittel" oder "verzehrfertiges Futtermittel" fallen auch Getränke sowie feste oder halbfeste verzehrfertige Lebensmittel oder Futtermittel. Als Beispiele seien Tiefkühlprodukte genannt, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln oder Futtermitteln.

Bevorzugte erfindungsgemäße oral konsumierbare Produkte (insbesondere Lebensmittel und Futtermittel) umfassen auch "Halbfertigwaren". Unter einer Halbfertigware ist im Zusammenhang des vorliegenden Textes ein oral konsumierbares Produkt zu verstehen, welches aufgrund eines sehr hohen Gehaltes an Aroma- und Geschmacksstoffen für die Verwendung als verzehrfertiges oral konsumierbares Produkt (insbesondere Lebensmittel oder Futtermittel) ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges oral konsumierbares Produkt (insbesondere Lebensmittel oder Futtermittel) überführt. Als Beispiel für Halbfertigwaren seien hier Tütensuppen, Backaromen und Puddingpulver genannt.

Zu erfindungsgemäßen oral konsumierbaren Produkten (insbesondere Lebensmitteln, Futtermitteln oder Arzneimitteln) zählen auch "Mundpflegeprodukte". Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind insbesondere Cremes, Gele, Pasten, Schäume, Emulsionen, Suspensionen, Aerosole, Sprays, wie auch Kapseln, Granulate, Pastillen, Tabletten, Bonbons oder Kaugummis, ohne dass diese Aufzählung für die Zwecke dieser Erfindung limitierend verstanden werden soll.

Bevorzugt umfasst ein erfindungsgemäßes oral konsumierbares Produkt (insbesondere Lebensmittel oder Futtermittel), eine oder mehrere der Ernährung oder dem Genuss dienende Zubereitungen. Hierunter fallen insbesondere (kalorienreduzierte) Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Dragees, Hart- und Weichkaramellen, Kaugummi), nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegartes Gemüse, eingekochtes Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitungen zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen und als Nahrungsergänzungsmittel vorliegen.

Besonders bevorzugt sind kalorienreduzierte Süßwaren (z.B. Müsliriegelprodukte, Fruchtgummi, Dragees, Hart- und Weichkaramellen, Kaugummi), nicht-alkoholische Getränke (z.B. Kakao, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Trockenmilchpulver, Molke, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Süßstoffzubereitungen, - tabletten oder -sachets, sonstige Zubereitungen zum Süßen oder Weißen von Getränken oder anderen Nahrungsmitteln.

Insbesondere bevorzugt sind kalorienreduzierte oder kalorienfreie Süßwaren (z.B. Müsliriegelprodukte, Fruchtgummi, Dragees, Hartkaramellen, Kaugummi), nicht-alkoholische Getränke (z.B. Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zuckerarme oder - freie Limonaden, isotonische Getränke, Nektare, Obst- und Gemüsesäfte, Frucht- oder Gemüsesaftzubereitungen), Instantgetränke (z.B. Instant-(Grün-, Schwarz, Rooibos, Kräuter)Tee-Getränke), Getreideprodukte (z.B. zuckerarme oder -freie Frühstückscerealien, Müsliriegel), Milchprodukte (z.B. fettreduzierte oder fettfreie Milchgetränke, Joghurt, Kefir, Molke, Buttermilch), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, oder daraus gefertigte Produkte und Mischungen mit Fruchtzubereitungen und fakultativ Aromen) oder Süßstoffzubereitungen, -tabletten oder -sachets.

Die Zubereitungen können in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die Halbfertigwaren dienen in der Regel zur Herstellung von der Ernährung oder dem Genuss dienenden gebrauchs- oder verzehrfertigen Zubereitungen.

Weitere Bestandteile einer der Ernährung oder dem Genuss dienenden verzehrfertigen Zubereitung bzw. Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosin-monophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, andere als die vorgenannten, synthetischen oder natürlichen oder naturidentischen Aromastoffe oder Riechstoffe sowie Geruchskorrigenzien.

Bevorzugt enthalten erfindungsgemäße oral konsumierbare Produkte (insbesondere Lebensmittel, Futtermittel und Arzneimittel), z.B. solche in Form von Zubereitungen oder Halbfertigwaren, eine Aromakomposition, um den Geschmack und/oder den Geruch abzurunden und zu verfeinern. Eine Zubereitung kann als Bestandteile einen festen Trägerstoff und eine Aromakomposition umfassen. Geeignete Aromakompositionen enthalten z.B. synthetische, natürliche oder naturidentische Aroma-, Riech- und Geschmacksstoffe, Reaktionsaromen, Raucharomen oder andere aromagebende Zubereitungen (z.B. Protein[teil]hydrolysate, bevorzugt Protein[teil]hydrolysate mit hohem Arginin-Gehalt, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier die Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Kardamom, Muskat, Piment, Senf und Senf-Produkte), gebratenen, hefigen, gesotteen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

Die Energiedichte eines oral konsumierbaren Produktes (insbesondere Lebensmittels, Futtermittels oder Arzneimittels) lässt sich zudem senken, indem energiereiche Inhaltsstoffe des oral konsumierbaren Produktes gegen Ersatzstoffe (z.B. kalorienarme Verdickungsmittel statt Fette, kalorienarme oder kalorienfreie Süßstoffe statt üblicher Zucker) ausgetauscht werden.

Demgemäß umfasst ein erfindungsgemäßes oral konsumierbares Produkt (insbesondere Lebensmittel, Futtermittel oder Arzneimittel) in einer bevorzugten Ausführungsform (a) einen, zwei oder mehrere Süßstoffe und/oder (b) ein, zwei oder mehrere Verdickungsmittel. Der Begriff "Süßstoffe" bezeichnet hierbei Stoffe mit einer relativen Süßkraft von zumindest 25, bezogen auf die Süßkraft von Saccharose (welches somit die Süßkraft 1 hat). Vorzugsweise sind in einem erfindungsgemäßen oral konsumierbaren Produkt (insbesondere Lebensmittel, Futtermittel oder Arzneimittel) (a) einzusetzende Süßstoffe nicht-kariogen und/oder besitzen einen Energiegehalt von maximal 5 kcal pro Gramm des oral konsumierbaren Produktes.

Erfindungsgemäß bevorzugt ist ein oral konsumierbares Produkt (insbesondere Lebensmittel oder Futtermittel), das durch Milchsäurebakterien verdickte Milch und/oder durch Milchsäurebakterien verdickte Sahne enthält und vorzugsweise ausgewählt ist aus der Gruppe bestehend aus oral konsumierbaren Produkten mit einem Fettgehalt von 4,0 Gew.-% oder weniger, bevorzugt von 1,5 Gew.-% oder weniger, besonders bevorzugt 0,5 Gew.-% oder weniger, jeweils bezogen auf das Gesamtgewicht des oral konsumierbaren Produktes,
und/oder
ausgewählt ist aus der Gruppe bestehend aus Joghurt, Kefir und Quark.

Vorzugsweise umfasst ein solches erfindungsgemäße oral konsumierbare Produkt (insbesondere Lebensmittel oder Futtermittel), das durch Milchsäurebakterien verdickte Milch und/oder durch Milchsäurebakterien verdickte Sahne enthält, einen Energiegehalt von nicht mehr als 150 kcal/100g des oral konsumierbaren Produktes, bevorzugt nicht mehr als 100 kcal/100g, besonders bevorzugt nicht mehr als 75 kcal/100g, besonders bevorzugt nicht mehr als 50 kcal/100g.

Ferner kann ein solches erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, zusätzlich Früchte und/oder Fruchtzubereitungen umfassen.

In einer bevorzugten Ausführungsform umfasst ein solches oral konsumierbares Produkt (insbesondere Lebensmittel oder Futtermittel), das durch Milchsäurebakterien verdickte Milch und/oder durch Milchsäurebakterien verdickte Sahne enthält,
(i) Zucker und/oder
(ii) Verdickungsmittel und/oder
(iii) Geliermittel und/oder
(iv) Süßungsmittel und/oder
(v) Aromen und/oder
(vi) Konservierungsstoffe.

"Zucker" ist im Rahmen des vorliegenden Textes (sofern nicht anders angegeben oder aus dem Kontext anders ersichtlich) der Sammelbegriff für alle süß schmeckenden Saccharide (Einfach- und Doppelzucker).

Vorteilhafterweise ist ein erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, ein oral konsumierbares Produkt, das ein Probiotikum enthält, wobei das Probiotikum vorzugsweise ausgewählt ist aus der Gruppe bestehend aus Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) und Lactobacillus rhamnosus (ATCC 53013).

Besonders bevorzugt ist ein erfindungsgemäßes oral konsumierbares Produkt (insbesondere Lebensmittel, Futtermittel oder Arzneimittel), wobei das oral konsumierbare Produkt ein Getränk ist,
wobei das Getränk vorzugsweise einen Zuckergehalt von 30 g/100 mL Getränk oder weniger, bevorzugt von 15 g/100 mL oder weniger, besonders bevorzugt 5 g/100 mL oder weniger aufweist, besonders bevorzugt kein Zucker enthält
und/oder
wobei das Getränk kein Ethanol oder maximal 0,1 Volumenprozent Ethanol enthält, bezogen auf das Volumen des Getränkes.

Im Rahmen der vorliegenden Erfindung sind erfindungsgemäße oral konsumierbare Produkte weniger bevorzugt, bei denen es sich um Ethanol enthaltende Getränke handelt.

Kein Ethanol bedeutet in der vorliegenden Erfindung, dass kein Ethanol zugesetzt wird und dass die Zubereitung weniger als 0,1 Vol.-%, bevorzugt weniger als 0,01 Vol.-% und besonders bevorzugt keine messbare Menge an Ethanol enthält.

Besonders bevorzugt sind erfindungsgemäße oral konsumierbare Produkte (vorzugsweise Lebensmittel, Futtermittel oder Arzneimittel), wobei es sich um ein kohlensäurehaltiges Getränk oder um ein kohlensäurefreies Getränk handelt.

Weitere Aspekte der vorliegenden Erfindung ergeben sich aus den nachfolgenden Beispielen und den beigefügten Ansprüchen.

### A. Kaugummis

Bei den bevorzugten oral konsumierbaren Produkten (insbesondere Lebensmittel, Futtermittel oder Arzneimittel) kann es sich auch um Kaugummis handeln. Diese Produkte enthalten typischerweise eine wasserunlösliche und eine wasserlösliche Komponente.

Die Kaugummibase ist dabei vorzugsweise ausgewählt aus der Gruppe bestehend aus "chewing gum" - oder "bubble gum" - Basen. Letztere sind weicher, damit sich damit auch Kaugummiblasen bilden lassen.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Erfindungsgemäß bevorzugte Kaugummibasen umfassen neben traditionell eingesetzten natürlichen Harzen oder dem Naturlatex Chicle Elastomere wie Polyvinylacetate (PVA), Polyethylene, (nieder- oder mittel-molekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336, US 5,601,858 oder US 6,986,709 beschrieben.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Daneben können erfindungsgemäß bevorzugt einzusetzende Kaugummibasen vorzugsweise weitere Bestandteile umfassen, wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

Erfindungsgemäße Kaugummis können allgemein Bestandteile umfassen, wie Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropion-säure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist unter anderem die Kombination der Aromen mit weiteren Stoffen, die kühlende, wärmende und/oder mundwässerndernde Eigenschaften aufweisen.

### B. Inhaltsstoffe für Lebensmittel, pharmazeutische Zubereitungen und oral konsumierbare Zubereitungen

Die Lebensmittelmittel, Futtermittel, pharmazeutischen Zubereitungen und oral konsumierbaren Zubereitungen der vorliegenden Erfindung können weitere Inhaltsstoffe aufweisen, wie z.B. Süßstoffe, Lebensmittelsäuren, Säureregulatoren, Verdickungsmittel und insbesondere Aromastoffe welche sowohl im Lebensmittelbereich, aber auch in pharmazeutischen Zubereitungen einsetzbar sein können, so dass hier keine starre Grenze zwischen den Inhaltstofflisten B und C gezogen werden kann. Die Inhaltstoffe werden demnach sinngemäß je nach Anwendung und Verwendung eingesetzt

### 1. Süßstoffe

Vorteilhafte Süßstoffe in einem bevorzugten erfindungsgemäßen oral konsumierbaren Produkt (insbesondere Lebensmittel, Futtermittel oder Arzneimittel) sind ausgewählt aus den folgenden Gruppen (a1) und (a2):
(a1) natürlich vorkommende Süßstoffe, vorzugsweise ausgewählt aus der Gruppe bestehend aus
(a1-1) Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physiologisch akzeptablen Salzen dieser Aminosäuren und/oder Proteine, insbesondere den Natrium-, Kalium-, Calcium oder Ammoniumsalzen;
(a1-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Dulcosiden und Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium oder Ammoniumsalzen;
(a1-3) Extrakte oder angereicherte Fraktionen der Extrakte, ausgewählt aus der Gruppe bestehend aus Thaumatococcus-Extrakten (Katemfestaude), Extrakten aus *Stevia* ssp. (insbesondere *Stevia rebaudiana*), Swingle-Extrakten (*Momordica* bzw. *Siratia grosvenorii,* Luo-Han-Guo), Extrakten aus *Glycerrhyzia* ssp. (insbesondere *Glycerrhyzia glabra*), Extrakten aus *Rubus* ssp. (insbesondere *Rubus suavissimus*), Citrus-Extrakten und Extrakten aus *Lippia dulcis*;
(a2) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen des Acesulfam, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate.

### 2. Säureregulatoren

Säureregulatoren sind Lebensmittelzusatzstoffe, die den Säuregrad oder die Basizität und damit den gewünschten pH-Wert eines Lebensmittels konstant halten. Es handelt sich meist um organische Säuren und deren Salze, Carbonate, seltener auch um anorganische Säuren und deren Salze. Der Zusatz eines Säureregulators verstärkt teils die Stabilität und Festigkeit des Lebensmittels, bewirkt eine erwünschte Ausfällung und verbessert die Wirkung von Konservierungsmitteln. Im Gegensatz zu Säuerungsmitteln werden sie nicht zur Geschmacksveränderung von Lebensmitteln benutzt. Ihre Wirkung beruht auf der Bildung eines Puffersystems im Lebensmittel, bei dem sich auf Zugabe von sauren oder basischen Stoffen der pH-Wert nicht oder nur geringfügig ändert. Beispiele sind:
Säuren im Sinne der Erfindung sind bevorzugt in Lebensmitteln zulässige Säuren, insbesondere die hier genannten:

| | |
|---|---|
| E 270 - | Milchsäure |
| E 290 - | Kohlendioxid |
| E 296 - | Apfelsäure |
| E 297 - | Fumarsäure |
| E 331 - | Natriumcitrat |
| E 332 - | Kaliumcitrat |
| E 333 - | Calciumcitrat |
| E 335 - | Natriumtartrat |
| E 336 - | Kaliumtartrat |
| E 337 - | Natrium-Kaliumtartrat |
| E 338 - | Phosphorsäure |
| E 353 - | Metaweinsäure |
| E 354 - | Calciumtartrat |
| E 363 - | Bernsteinsäure |
| E 380 - | Triammoniumcitrat |
| E 513 - | Schwefelsäure |
| E 575 - | Glucono-delta-Lacton |
| E 170 - | Calciumcarbonat |
| E 260-263 | Essigsäure und Acetate |
| E 325-327 | Lactate (Milchsäure) |
| E 330-333 | Citronensäure und Citrate |
| E 334-337 | Weinsäure und Tartrate |
| E 339-341 | Orthophosphate |
| E 350-352 | Malate (Äpfelsäure) |
| E 450-452 | Di-, Tri- und Polyphosphate |
| E 500-504 | Carbonate (Kohlensäure) |
| E 507 - | Salzsäure und Chloride |
| E 513-517 | Schwefelsäure und Sulfate |
| E 524-528 | Hydroxide |
| E 529-530 | Oxide |
| E 355-357 | Adipinsäure und Adipate |
| E 574-578 | Gluconsäure und Gluconate |

### 3. Verdickungsmittel

Verdickungsmittel sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder lonenbeziehungen miteinander interagieren können. Extremfälle von Dickungsmitteln sind Schichtsilikate (Bentonite, Hectorite) oder hydratisierte SiO₂-Partikel, die als Teilchen dispergiert vorliegen und in ihrer festkörperartigen Struktur Wasser binden können bzw. aufgrund der beschriebenen Wechselwirkungen miteinander interagieren können. Beispiele sind:

| | |
|---|---|
| E 400 - | Alginsäure |
| E 401 - | Natriumalginat |
| E 402 - | Kaliumalginat |
| E 403 - | Ammoniumalginat |
| E 404 - | Calciumalginat |
| E 405 - | Propylenglycolalginat |
| E 406 - | Agar Agar |
| E 407 - | Carrgeen, Furcelleran |
| E 407 - | Johannisbrotkernmehl |
| E 412 - | Guarkernmehl |
| E 413 - | Traganth |
| E 414 - | Gummi arabicum |
| E 415 - | Xanthan |
| E 416 - | Karaya (Indischer Traganth) |
| E 417 - | Tarakernmehl (Peruanisches Johannisbrotkernmehl) |
| E 418 - | Gellan |
| E 440 - | Pektin, Opekta |
| E 440ii - | Amidiertes Pektin |
| E 460 - | Mikrokristalline Cellulose, Cellulosepulver |
| E 461 - | Methylcellulose |
| E 462 - | Ethylcellulose |
| E 463 - | Hydroxypropylcellulose |
| E 465 - | Methylethylcellulose |
| E 466 - | Carboxymethylcellulose, Natriumcarboxymethylcellulose |

### 4. Aromastoffe

Geeignete Aromastoffe sind vorzugsweise eine sensorisch wirksame Komponente und werden bevorzugt eingesetzt in einer Konzentration, die größer ist als sein Reizschwellenwert. Bevorzugte Aromastoffe, die Bestandteil der Stoffmischung sein können, finden sich z.B. in H. Surburg, J. Panten, Common Fragrance and Flavor Materials, 5th. Ed., Wiley-VCH, Weinheim 2006.

Die Aromastoffe können in Form von Aromakompositionen eingesetzt werden, die wiederum in Form von Reaktionsaromen (Maillard-Produkte) und/oder Extrakten bzw. ätherischen Ölen von Pflanzen oder Pflanzenteilen bzw. Fraktionen davon eingesetzt werden können.

Die erfindungsgemäßen Lebensmittel oder pharmazeutische Zubereitungen der vorliegenden Erfindung können einen oder mehrere Aromastoffe enthalten. Typische Beispiele umfassen: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

### 5. Vitamine

In einer weiteren Ausführungsform der vorliegenden Erfindung können Lebensmittel oder pharmazeutische Zubereitungen weitere fakultative Gruppe von Zusatzstoffen Vitamine enthalten. Vitamine verfügen über unterschiedlichste biochemische Wirkungsweisen. Einige wirken ähnlich wie Hormone und regulieren den Mineralmetabolismus (z.B. Vitamin D), oder wirken auf das Wachstum von Zellen und Gewebe sowie die Zelldifferenzierung (z.B. einige Formen des Vitamin A). Andere stellen Antioxidantien dar (z.B. Vitamin E und unter bestimmten Umständen auch Vitamin C). Die größte Zahl von Vitaminen (z.B. die B-Vitamine) stellen Vorstufen für enzymatische Co-Faktoren dar, die Enzyme dabei unterstützen, bestimmte Prozesse im Metabolismus zu katalysieren. In diesem Zusammenhang können Vitamin mitunter eng an die Enzyme gebunden sein, beispielsweise als Teil der prostetischen Gruppe: ein Beispiel hierfür ist Biotin, das ein Teil des Enzyms ist, welches für den Aufbau von Fettsäuren verantwortlich ist. Vitamine können andererseits auch weniger stark gebunden sein und dann als Co-Katalysatoren wirken, beispielsweise als Gruppen, die sich leicht abspalten lassen und chemische Gruppen oder Elektronen zwischen den Molekülen transportieren. So transportiert beispielsweise Folsäure Methyl-, Formyl- und Methylengruppen in die Zelle. Obwohl ihre Unterstützung in Enzym-Substrat-Reaktionen wohl bekannt ist, sind auch ihre übrigen Eigenschaften für den Körper von großer Bedeutung.

Im Rahmen der vorliegenden Erfindung kommen als Vitamine Stoffe in Betracht, die ausgewählt sind aus der Gruppe bestehend aus
- Vitamin A (Retinol, Retinal, Betakarotin),
- Vitamin B₁ (Thiamin),
- Vitamin B₂ (Rioflavin),
- Vitamin B₃ (Niacin, Niacinamid),
- Vitamin B₅ (Panthothensäure),
- Vitamin B₆ (Pyridoxin, Pyridoxamin, Paridoxal),
- Vitamin B₇ (Biotin),
- Vitamin B₉ (Folsäure, Folinsäure),
- Vitamin B₁₂ (Cyanobalamin, Hydroxycobalmin, Methylcobalmin),
- Vitamin C (Ascorbinsäure),
- Vitamin D (Cholecalciferol),
- Vitamin E (Tocopherole, Tocotrienole) und
- Vitamin K (Phyllochinon, Menachinon).

Die bevorzugten Vitamine sind neben der Ascorbinsäure die Gruppe der Tocopherole.

### 6. Prebiotische Stoffe

In einer weiteren Ausgestaltung der Erfindung können Lebensmittel oder pharmazeutische Zubereitungen der vorliegenden Erfindung des Weiteren Prebiotische Stoffe ("Prebiotics") enthalten, die die Gruppe H bilden. Prebiotics werden als unverdauliche Nahrungsbestandteile definiert, deren Verabreichung das Wachstum oder die Aktivität einer Reihe nützlicher Bakterien im Dickdarm stimuliert. Die Zugabe von prebiotischen Verbindungen verbessert die Stabilität der Anthocyanine gegenüber Abbauprozessen im Darmtrakt. Im Folgenden werden verschiedene Stoffe, insbesondere Kohlenhydrate, die als Prebiotics im Sinne der Erfindung besonders bevorzugt sind.

**Fructooligosaccharide.** Fructooligosaccharide oder abgekürzt FOS umfassen insbesondere kurzkettige Vertreter mit 3 bis 5 Kohlenstoffatomen, wie beispielsweise D-Fructose und D-Glucose. FOS, auch als Neozucker bezeichnet, werden kommerziell auf Basis von Saccharose und dem aus Pilzen gewonnenen Enzym Fructosyltransferase hergestellt. FOS unterstützen insbesondere das Wachstum von Bifidobakterien im Darm und werden vor allem in den USA zusammen mit probiotischen Bakterien in verschiedenen funktionalisierten Lebensmitteln vermarktet.

**Inuline.** Inuline zählen zu einer Gruppe von natürlich vorkommenden Fructose enthaltenden Oligosachhariden. Sie gehören zu einer Klasse von Kohlenhydraten, die als Fructane bezeichnet werden. Ihre Gewinnung erfolgt aus den Wurzeln der Chicoree-Pflanze (Cichorium intybus) oder so genannten Jerusalem-Artischocken. Inuline bestehen überwiegend aus Fructoseeinheiten und weisen typisch eine Glucoseeinheit als Endgruppe auf. Die Fructoseeinheiten sind dabei miteinander über eine beta-(2-1)glykosidische Bindung verknüpft. Der mittlere Polymerisationsgrad von Inulinen, die als Prebiotics im nahrungsmittelbereich Anwendung finden, liegt bei 10 bis 12. Inuline stimulieren ebenfalls das Wachstum von Bifidobakterien im Dickdarm.

**Isomaltooligosaccharide.** Bei dieser Gruppe handelt es sich um eine Mischung von alpha-D-verknüpften Glucoseoligomeren, einschließlich Isomaltose, Panose, isomaltotetraose, Isoma-Itopentaose, Nigerose, Kojibiose, Isopanose und höherer verzweigter Oligosaccharide. Isoma-Itooligosaccharide werden über verschiedene enzymatische Wege hergestellt. Sie stimulieren ebenfalls das Wachstum von Bifidobakterien und Lactobacillen im Dickdarm. Isomaltooligosaccharide werden speziell in Japan als Nahrungsmittelzusatzstoffe in funktionalisierten Lebensmitteln eingesetzt. Inzwischen finden sie auch in den USA Verbreitung.

**Lactilol.** Lactilol ist das Disaccharid der Lactulose. Seine medizinische Anwendung findet gegen Verstopfung und bei häpatischer Enzephalopathie. In Japan wird Lactilol als prebiotic eingesetzt. Es widersteht dem Abbau im oberen Verdauungstrakt, wird aber durch verschiedene Darmbakterien fermentiert, was zu einem Anstieg der Biomasse an Bifidobakterien und Lactobacillen im Darm führt. Lactilol ist auch unter der chemischen Bezeichnung 4-0-(beta-D-galactopyranosyl)-D-glucitol bekannt. Der medizinische Anwendungsbereich von Lactilol in den USA ist wegen fehlender Studien beschränkt; in Europa wird es vorzugsweise als Süßstoff eingesetzt.

**Lactosucrose.** Lactosucrose ist ein Trisaccharid, das sich aus D-Galactose, D-Glucose und D-Fructose aufbaut. Lactosucrose wird durch enzymatischen Transfer des Galactosylrestes in der Lactose auf die Sucrose hergestellt. Es wird weder im Magen noch im oberen Teil des Darmtraktes abgebaut und wird ausschließlich von Bifidobakterien zu Wachstum konsumiert. Unter physiologischen Gesichtspunkten wirkt Lactosucrose als Stimulator für das Wachstum der Darmflora. Lactosucrose ist ebenfalls bekannt als 4G-beta-D-galactosucrose. Es ist in Japan als Nahrungsmittelzusatzstoff und als Bestandteil von funktionalisierten lebensmittel weit verbreitet, insbesondere auch als Zusatzstoff für Joghurts. Lactosucrose wird derzeit auch in den USA für einen ähnlichen Anwendungszweck getestet.

**Lactulose.** Lactulose ist ein halbsynthetisches Disaccharid aus D-Lactose und D-Fructose. Die Zucker sind über eine beta-glykosidische Bindung verknüpft, was sie resistent gegen Hydrolyse durch Verdauungsenzyme macht. Stattdessen wird Lactulose durch eine beschränkte Anzahl von Darmbakterien fermentiert, was zu einem Wachstum insbesondere von Lactobacillen und Bifidobakterien führt. Lactulose stellt in den USA ein verschreibungspflichtiges Medikament gegen Verstopfung und hepatische Enzephalopathie dar. In Japan hingegen wird es als Nahrungsmittelzusatzstoff und Bestandteil von funktionalisierten Lebensmitteln frei verkauft.

**Pyrodextrine.** Pyrodextrine umfassen eine Mischung von glucoseenthaltenden Oligosacchariden, die bei der Hydrolyse von Stärke gebildet werden. Pyrodextrine fördern die Proliferation von Bifidobakterien im Dickdarm. Auch sie werden im oberen Darmbereich nicht abgebaut.

**Sojaoligosaccharide.** Bei dieser Gruppe handelt es sich um Oligosaccharide, die im Wesentlichen nur in Sojabohnen und darüber noch in anderen Bohnen sowie Erbsen zu finden sind. Die beiden maßgeblichen Vertreter sind das Trisaccharid Raffinose und das Tetrasaccharid Stachyose. Raffinose setzt sich aus jeweils einem Molekül D-Galactose, D-Glucose und D-Fructose zusammen. Stachyose besteht aus zwei Molekülen D-Galactose sowie jeweils einem Molekül D-Glucose und D-Fructose. Sojaoligosacccharide stimulieren das Wachstum von Bifidobakterien im Dickdarm und werden in Japan bereits als Nahrungsmittelzusatzstoffe sowie in funktionalisierten Lebensmitteln eingesetzt. In den USA werden sie für diese Anwendung derzeit getestet.

**Transgalactooligosaccharide.** Transgalactooligosaccharide (TOS) stellen Mischungen von Oligosacchariden auf Basis D-Glucose und D-Galactose dar. TOS werden ausgehend von D-Lactose mit Hilfe des Enzyms Betaglucosidase aus Aspergillus oryzae hergestellt. Wie viele andere Prebiotics sind auch TOS im Dünndarm stabil und stimulieren das Wachstum von Bifidobakterien im Dickdarm. TOS werden sowohl bereits in Europa als auch in Japan als Nahrungsmittelzusatzstoffe vermarktet.

**Xylooligosaccharide.** Xylooligosaccharide enthalten beta-1,4-verknüpfte Xyloseeinheiten. Der Polymerisationsgrad der Xylooligosaccharide liegt zwischen 2 und 4. Sie werden durch enzymatische Hydrolyse des Polysaccharids Xylan erhalten. Sie werden bereits in Japan als Nahrungsmittelzusatzstoffe vermarktet, in den USA befinden sie sich noch in der Testphase.

**Biopolymere.** Geeignete Biopolymere, die ebenfalls als Prebiotics in Betracht kommen, wie beispielsweise Beta-Glucane, zeichnen sich dadurch aus, dass sie auf pflanzlicher Basis hergestellt werden, beispielsweise kommen als Rohstoffquellen Cerealien wie Hafer und Gerste, aber auch Pilze, Hefen und Bakterien in Frage. Außerdem geeignet sind mikrobiell hergestellte Zellwandsuspensionen oder ganze Zellen mit hohem Beta-Glucan Gehalt. Restliche Anteile an Monomeren weisen 1-3 und 1-4 oder 1-3 und 1-6 Verknüpfungen auf, wobei der Gehalt stark variieren kann. Vorzugsweise werden Beta-Glucane auf Basis von Hefen, insbesondere Saccharomyces, speziell Saccharomyces cerevisiae, erhalten. Andere geeignete Biopolymere sind Chitin und Chitinderivate, insbesondere Oligoglucosamin und Chitosan, das ein typisches Hydrokolloid darstellt.

**Galactooligosaccharide (GOS).** Galacto-Oligosaccharide werden durch die enzymatische Umwandlung von Lactose, einer Komponente von Rindermilch, erzeugt. GOS umfassen im Allgemeinen eine Kette von Galactose-Einheiten, die durch aufeinanderfolgende Transgalactosylierung-Reaktionen gebildet werden, und die eine terminale Glucoseeinheit aufweisen. Terminale Glucoseeinheiten werden zumeist durch eine frühzeitige Hydrolyse von GOS gebildet. Der Polymerisationsgrad der GOS kann ziemlich stark schwanken und reicht von 2 bis 8 Monomereinheiten. Eine Reihe von Faktoren bestimmt dabei den Aufbau und die Reihenfolge Monomereinheiten: die Enzym-Quelle, das Ausgangsmaterial (Lactose-Konzentration und Ursprung der Lactose), die am Prozess beteiligten Enzyme, Bedingungen bei der Verarbeitung und die Zusammensetzung des Mediums.

### 7. Probiotische Mikroorganismen

Probiotische Mikroorganismen, auch als "Probiotics" bezeichnet, stellen lebende Mikroorganismen dar, die für den Wirt nützliche Eigenschaften besitzen. Gemäß der Definition der FAO/WHO handelt es sich um "lebende Mikroorganismen, die bei angemessener Dosierung dem Wirt einen Gesundheitsvorteil vermitteln". Milchsäurebakterien (LAB) und Bifidobakterien stellen die bekanntesten Probiotics dar; es können aber auch verschiedene Hefen und Bazillen Verwendung finden. Probiotics werden üblicherweise als Bestandteil von fermentierten Nahrungsmitteln aufgenommen, denen man spezielle Lebendkulturen zugesetzt hat, wie z.B. Joghurt, Sojajoghurt oder andere probiotische Nahrungsmittel. Darüber hinaus sind auch Tabletten, kapseln, Pulver und Sachets erhältlich, die die Mikroorganismen in gefriergetrockneter Form enthalten. Tabelle A gibt einen Überblick über handelsübliche Probiotics und den zugehörigen Auslobungen zur Gesundheit, die im Sinne der vorliegenden Erfindung eingesetzt werden können.

**Tabelle A: Probiotische Stoffe**

| **Stamm** | **Bezeichnung** | **Hersteller** | **Auslobung** |
|---|---|---|---|
| *Bacillus coagulans* GBI-30, 6086 | GanedenBC | Ganeden Biotech | Steigert die Immunantwort bei viraler Infektion |
| *Bifidobacterium animalis* subsp. *lactis* BB-12 | Probio-Tec Bifidobacterium BB-12 | Chr. Hansen | Klinische Studien am Menschen haben gezeigt, dass BB-12 alleine oder in Kombination das gastrotestinale System positiv beeinflusst. |
| *Bifidobacterium infantis* 35624 | Align | Procter & Gamble | In einer vorläufigen Studie wurde gezeigt, dass das Bakterium abdominale Schmerzen verringern kann. |
| *Lactobacillus acidophilus* NCFM | | Danisco | Aus einer Studie geht hervor, dass die Nebeneffekte von antibiotischen Behandlungen verringert werden |
| *Lactobacillus paracasei* St11 (or NCC2461) | | | |
| *Lactobacillus johnsonii* La1 (= Lactobacillus LC1, *Lactobacillus johnsonii* NCC533) | | Nestlé | Verringert Gastritisbeschwerden und vemindert Entzündungen |
| *Lactobacillus plantarum* 299v | GoodBelly/ Pro-Viva/ProbiMage | Probi | Könnte IBS Symptome verbessern; jedoch noch mehr S tu dien erforderlich. |
| *Lactobacillus reuten* American Type Culture Collection\|ATTC 55730 (*Lactobacillus reuteri* SD2112) | | BioGaia | Erste Anzeichen für eine Wirksamkeit gegen Gangivitis, Fieber bei Kindern und Verminderung der Krankheitstage bei Erwachsenen. |
| *Lactobacillus reuteri* Protectis (DSM 17938, daughter strain of ATCC 55730) | | | |
| *Lactobacillus reuteri* Protectis (DSM 17938, daughter strain of ATCC 55730) | | | |
| *Saccharomyces boulardii* | DiarSafe and others | Wren Laboratories | Beschränkter Nachweis bei der Behandlung von akuten Durchfallerkrankungen. |
| *Lactobacillus rhamnosus* GR-1 & *Lactobacillus reuteri* RC-14 | Bion Flore Intime/ Jarrow Fem-Dophilus | Chr. Hansen | In einer Studie Nachweis der Wirksamkeit gegen Vaginitis. |
| *Lactobacillus acidophilus* NCFM & *Bifidobacterium bifidum* BB-12 | Florajen3 | American Lifeline, Inc | Erste Hinweise auf Wirksamkeit gegen CDAD |
| *Lactobacillus acidophilus* CL1285 & *Lactobacillus casei* LBC80R | Bio-K+ CL1285 | Bio-K+ International | Hinweise auf Verbesserung bei der Verdauung, speziell im Hinblick auf Lactoseintoleranz. |
| *Lactobacillus plantarum* HEAL 9 & *Lactobacillus paracasei* 8700:2 | Bravo Friscus/ ProbiFrisk | Probi | Derzeit laufen Untersuchungen zur Wirksamkeit gegen Erkältungs-erkrankungen. |

Im Folgenden werden zwei weiteren Formen von Milchsäurebakterien genannt, die ebenfalls als Probiotics eingesetzt werden können:
- *Lactobacillus bulgaricus;*
- *Streptococcus thermophilus;*

Auch spezielle fermentierte Produkte auf Basis solcher Milchsäurebakterien sind einsetzbar:
- Mixed Pickles
- Fermented bean paste such as tempeh, miso and doenjang;
- Kefir;
- Buttermilch
- Kimchi;
- Pao cai;
- Sojasoße;
- Zha cai.

### 8. Geschmacksverstärker und Aromastoffe mit geschmacksmodifizierenden Eigenschaften

Die Lebensmittel oder pharmazeutische Zubereitungen der vorliegenden Erfindung können des Weiteren zusätzliche Aromastoffe zum Verstärken eines salzigen, gegebenenfalls leicht sauren und/oder Umami-Geschmackseindrucks enthalten. Hierbei bevorzugt sind salzig schmeckende Verbindungen und salzverstärkende Verbindungen. Bevorzugte Verbindungen sind in der WO 2007/045566 offenbart. Ferner bevorzugt sind Umami-Verbindungen wie in der WO 2008/046895 und EP 1 989 944 beschrieben sind.

Weiterhin können Lebensmittel oder pharmazeutische Zubereitungen der vorliegenden Erfindung auch Aromastoffe zur Maskierung von bitteren und/oder adstringierenden Geschmackseindrücken umfassen (Geschmackskorrigentien). Die (weiteren) Geschmackskorrigenzien werden z. B. aus der folgenden Liste ausgewählt: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxyben-zyl)amid-Mono-natriumsalz, 2,4-Dihydroxybenzoesäure-N-2-(4-hydroxy-3-methoxyphenyl)-ethylamid, 2,4-Dihydroxybenzoesäure-N-(4-hydroxy-3-ethoxybenzyl)amid, 2,4-Dihydroxy-benzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskie-rende Hydroxydeoxybenzoine z.B. gemäß WO 2006/106023 (z.B. 2-(4-Hydroxy-3-methoxyphenyl)-1-(2,4,6-tri-hydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxy-phenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen gemäß PCT/EP 2006/067120 Diacetyltrimere gemäß WO 2006/058893, Gemische von Molkeproteinen mit Lecithinen und/oder bittermaskierende Substanzen wie Gingerdione gemäß WO 2007/003527, 4-Hydroxydihydrochalkone nach EP 1,972,203, Dihydochalkone nacj EP 2,353,403, Alkamide wie Pellitorin nach EP 2,058,297, Hydroxyflavane nach EP 2,253,226, Rubus suavissimus-Extrakte nach EP 2,386,211, Vanillylignane nach EP 2,517,574, Neoisoflavonoids nach EP 2,570,035, Decadienoylaminosäuren nach EP 2,597,082 oder heterocyclische Neoflavonoide nach EP 2,725,026.

Bevorzugte Aromastoffe sind solche, die einen süßen Geruchseindruck verursachen, wobei der oder die weiteren Aromastoffe, die einen süßen Geruchseindruck verursachen, bevorzugt ausgewählt sind aus der Gruppe bestehend aus:
Vanillin, Ethylvanillin, Ethylvanillinisobutyrat (= 3 Ethoxy-4-isobutyryloxybenzaldehyd), Furaneol (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al, 4-Hydroxyzimtsäure, 4-Methoxy-3-hydroxyzimtsäure, 3-Methoxy-4-hydroxyzimtsäure, 2-Hydroxyzimtsäure, 2,4-Dihydroxybenzoesäure, 3-Hydroxybenzoesäure, 3,4-Dihydroxybenzoesäure, Vanillinsäure, Homovanillinsäure, Vanillomandelsäure und Phenylacetaldehyd.

Besonders bevorzugt sind Aromastoffe, die den süßen Geschmack positiv beeinflussen können, ohne selber eine starke Süßwirkung zu zeigen, die bevorzugt ausgewählt werden aus der Gruppe bestehend aus: Hesperetin nach WO 2007/014879 A1, Hydroxyphenylalkadione nach WO 2007/003527 A1, 4-Hydroxychalkone nach WO 2007/107596 A1 und EP 1 972 203, Propenylphenylglycoside (Chavicoglycoside) nach EP 1 955 601 A1, Phyllodulcin (oder Extrakte enthaltend Phyllodulcin) nach EP 2 298 084, Balansin A oder Balansin B (oder Extrakte enthaltend Balansin A oder B) nach WO 2012/164062, Hydroxyflavane nach EP 2 253 226, 1-(2,4-Dihydroxy-phenyl)-3-(3-hydroxy-4-methoxy-phenyl)-propan-1-on nach EP 2 353 403, enzymatisch behandelten Rubusosiden nach Anmeldenummer EP 14172306.4 oder Neoisoflavonoiden nach EP 2 570 036.

### 9. Antioxidantien

In der Lebensmittelindustrie werden sowohl natürliche als auch künstliche Antioxidationsmittel verwendet. Natürliche und künstliche Antioxidantien unterscheiden sich in erster Linie dadurch, dass erstere natürlich in der Nahrung vorkommen und letztere künstlich hergestellt werden. So werden natürliche Antioxidationsmittel, so sie als Lebensmittelzusatzstoff eingesetzt werden sollen, beispielsweise aus Pflanzenölen gewonnen. Vitamin E - auch als Tocopherol bekannt - wird beispielsweise häufig aus Sojaöl hergestellt. Synthetische Antioxidantien wie das Propylgallat, das Octylgallat und das Dodecylgallat werden dagegen durch chemische Synthese gewonnen. Die Gallate können bei empfindlichen Personen Allergien auslösen. Weitere einsetzbare Antioxidantien in Zusammensetzungen der vorliegenden Erfindung sind: Schwefeldioxid, E 220 Sulfite Natriumsulfit, E 221 Natriumhydrogensulfit, E 222 Natriumdisulfit, E 223 Kaliumdisulfit, E 224 Kalziumsulfit, E 226 Kalziumhydrogensulfit, E 227 Kaliumhydrogensulfit, E 228 Milchsäure, E 270 Ascorbinsäure, E 300 Natrium-L-Ascorbat, E 301 Calcium-L-Ascorbat, E 302 Ascorbinsäureester, E 304 Tocopherol, E 306 Alpha-Tocopherol, E 307 Gamma-Tocopherol, E 308 Delta-Tocopherol, E 309 Propylgallat, E 310 Octygallat, E 311 Dodecylgallat, E 312 Isoascorbinsäure, E 315 Natriumisoascorbat, E 316 tertiär-Butylhydrochinon (TBHQ), E 319 Butylhydroxianisol, E 320 Butylhydroxitoluol, E 321 Lecithin, E 322 Citronensäure, E 330 Salze der Zitronensäure (E 331 & E 332) Natriumzitrat, E 331 Kaliumzitrat, E 332 Calcium-Dinatrium-EDTA, E 385 Diphosphate, E 450 Dinatriumdiphosphat, E 450a Trinatriumdiphosphat, E 450b Tetranatriumdiphosphat, E 450c Dikaliumdiphosphat, E 450d Trekaliumdiphosphat, E 450e Dikalziumdiphosphat, E 450f Kalziumdihydrogendiphosphst, E 450g Triphosphate, E 451 Pentanatriumtriphosphat, E 451a Pentakaliumtriphosphat, E 451b Polyphosphat, E 452 Natriumpolyphosphat, E 452a Kaliumpolyphosphat, E 452b Natriumkalziumpolyphosphat, E 452c Kalziumpolyphosphat, E 452d Zinn-II-Chlorid, E 512.

### 10. Emulgatoren

Emulgatoren zeichnen sich durch die wichtige Eigenschaft aus, sowohl in Wasser als auch in Fett löslich zu sein. Emulgatoren bestehen meist aus einem fettlöslichen und einem wasserlöslichen Teil. Sie kommen immer dann zum Einsatz, wenn Wasser und Öl zu einer beständigen, homogenen Vermischung gebracht werden sollen. Geeignete Emulgatoren, die in der lebensmittelverarbeitenden Industrie verwendet werden sind ausgewählt aus: Ascorbylpalmitat (E 304) Lezithin (E 322) Phosphorsäure (E 338) Natriumphosphat (E 339) Kaliumphosphat (E 340) Kalziumphosphat (E 341) Magnesiumorthophosphat (E 343) Propylenglykolalginat (E 405) Polyoxyethylen(8)stearat (E 430) Polyoxyethylenstearat (E 431) Ammoniumphosphatide (E 442) Natriumphosphat und Kaliumphosphat (E 450) Natriumsalze der Speisefettsäuren (E 470 a) Mono- und Diglyceride von Speisefettsäuren (E 471) Essigsäuremonoglyceride (E 472 a) Milchsäuremonoglyceride (E 472 b) Zitronensäuremonoglyceride (E 472 c) Weinsäuremonoglyceride (E 472 d) Diacetylweinsäuremonoglyceride (E 472 e) Zuckerester von Speisefettsäuren (E 473) Zuckerglyceride (E 474) Polyglyceride von Speisefettsäuren (E 475) Polyglycerin-Polyricinoleat (E 476) Propylenglykolester von Speisefettsäuren (E 477) Natriumstearoyllaktylat (E 481) Calciumstearoyl-2-lactylat (E 482) Stearyltartrat (E 483) Sorbitanmonostearat (E 491) Stearinsäure (E 570).

### 11. Lebensmittelfarbstoffe

Lebensmittelfarbstoffe oder kurz Farbstoffe sind Lebensmittelzusatzstoffe zum Färben von Lebensmittel. Farbstoffe werden in die Gruppen der natürlichen Farbstoffe und synthetischen Farbstoffe unterteilt. Die naturidentischen Farbstoffe sind ebenfalls synthetischen Ursprungs. Die naturidentischen Farbstoffe sind synthetische Nachbildungen von in der Natur vorkommenden, färbenden Substanzen. Geeignete Farbstoffe für den Einsatz in der vorliegenden Zusammensetzung sind ausgewählt aus: Kurkumin, E 100 Riboflavin, Lactoflavin, Laktoflavin, Vitamin B2, E 101 Tartrazin, E 102 Chinolingelb, E 104 Gelborange S, Gelborange RGL, E 110 Cochenille, Karminsäure, echtes Karmin, E 120 Azorubin, Carmoisin, E 122 Amaranth, E 123 Cochenillerot A, Ponceau 4 R, Victoriascharlach 4 R, E 124 Erythrosin, E 127 Allurarot AC, E 129 Patentblau V, E 131 Indigotin, Indigo-Karmin, E 132 Brillantblau FCF, Patentblau AE, Amidoblau AE, E 133 Chlorophylle, Chlorophylline, E 140 Kupferkomplexe der Chlorophylle, Kupfer-Chlorophyllin-Komple, E 141 Brillantsäuregrün, Grün S, E 142 Zuckerkulör, Zuckercouleur, E 150 a Sulfitlaugen-Zuckerkulör, E 150 b Ammoniak-Zuckerkulör, E 150 c Ammoniumsulfit-Zuckerkulör, E 150 d Brillantschwarz FCF, Brillantschwarz PN, Schwarz PN, E 151 Pflanzenkohle, E 153 Braun FK, E 154 Braun HT, E 155 Carotin, Karotin, E 160 a Annatto, Bixin, Norbixin, E 160 b Capsanthin, Capsorubin, E 160 c Lycopin, E 160 d Beta-apo-8'-Carotinal, Apocarotinal, Beta-Apocarotinal, E 160 e Beta-apo-8'-Carotinsäure-Ethylester (C30), Apocarotinester, Beta-Carotinsäureester, E 160 f Lutein, Xanthophyll, E 161 b Canthaxanthin, E 161 g Betanin, Betenrot, E 162 Anthocyane, E 163 Calciumcarbonat, E 170 Titandioxid, E 171 Eisenoxide, Eisenhydroxide, E 172 Aluminium, E 173 Silber, E 174 Gold, E 175 Litholrubin BK, Rubinpigment BK, E 180.

### 12. Kühlwirkstoffe

Geeignete Kühlwirkstoffe sind beispielsweise Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

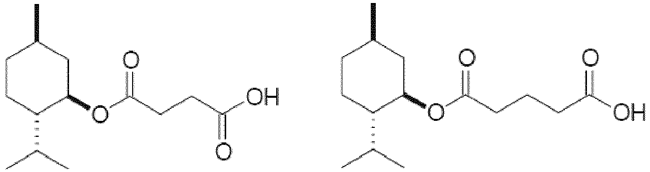

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat® MGA, Frescolat® ML, Frecolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

Vorteilhafte Trägerstoffe sind (vorzugsweise sprühgetrockneten) hierbei Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine. Bevorzugte Trägerstoffe sind Siliciumdioxid, Gummi Arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Maisbasierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

### VERKAPSELUNG

Stoffmischungen und Zubereitungen der vorliegenden Erfindung, welche Hydroxyflavone der Formel (I) umfassen, können je nach Bedarf auch verkapselt werden. Üblicherweise wird mit Hilfe von Festen Überzugsmaterialien verkapselt, wie beispielsweise Stärken, einschließlich deren Abbauprodukten sowie chemisch oder physikalisch erzeugten Derivaten (insbesondere Dextrine und Maltodextrine), Gelatine, Gummi Arabicum, Agar-Agar, Ghatti Gum, Gellan Gum, modifizierte und nicht-modifizierte Cellulosen, Pullulan, Curdlan, Carrageenane, Alginsäure, Alginate, Pektin, Inulin, Xanthan Gum und Mischungen von zwei oder mehreren dieser Substanzen.

Das feste Verkapselungsmaterial ist vorzugsweise eine Gelatine (insbesondere Schweine-, Rind-, Geflügel- und/oder Fischgelatine), wobei diese vorzugsweise einen Schwellfaktor von größer oder gleich 20, vorzugsweise von größer oder gleich 24 aufweist. Ebenfalls bevorzugt sind Maltodextrine (insbesondere auf Basis von Getreide, speziell Mais, Weizen, Tapioka oder Kartoffeln), die vorzugsweise DE-Werte im Bereich von 10 bis 20 aufweisen. Weiterhin bevorzugt sind Cellulosen (z.B. Celluloseether), Alginate (z.B. Natriumalginat), Carrageenan (z.B. beta-, jota-, lambda- und/oder kappa-Carrageenan), Gummi Arabicum, Curdlan und/oder Agar Agar.

Unter diesen Stoffen ist Gelatine besonders bevorzugt, da sie gut verfügbar ist und mit unterschiedlichen Schwellfaktoren bezogen werden kann. Ganz besonders bevorzugt, insbesondere für orale Anwendungen, sind nahtlose Gelatine- oder Alginatkapseln, deren Hülle sich im Mund oder beim Kauen sehr rasch auflöst oder aufbricht. Entsprechende Kapseln werden beispielsweise in den folgenden Schriften ausführlich EP 0389700 A1**,** US 4,251,195**,** US 6,214,376**,** WO 2003 055587 oder WO 2004 050069 A1**.**

Die Kapseln können alternativ auch Mikrokapseln darstellen. Unter den Begriffen "Mikrokapsel" oder "Nanokapsel" werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.
Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).
Chitosanmikrokapseln und Verfahren zu ihrer Herstellung sind aus dem Stand der Technik hinlänglich bekannt **[**WO 01/01926**,** WO 01/01927**,** WO 01/01928**,** WO 01/01929**].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a) aus Gelbildnern, kationischen Polymeren und Wirkstoffen eine Matrix zubereitet,
(b) gegebenenfalls die Matrix in einer Ölphase dispergiert,
(c) die dispergierte Matrix mit wässrigen Lösungen anionischer Polymere behandelt und gegebenenfalls dabei die Ölphase entfernt.
Die Schritte (a) und (c) sind dabei insofern austauschbar, als man anstelle der kationischen Polymeren in Schritt (a) anionische Polymere einsetzt und umgekehrt.

Man kann die Kapseln auch erzeugen, indem man den Wirkstoff abwechselnd mit Schichten aus unterschiedlich geladenen Polyelektrolyten einhüllt (layer-by-layer-Technologie). In diesem Zusammenhang sei auf das Europäische Patent EP 1064088 B1 (Max-Planck Gesellschaft) verwiesen.
Stoffmischungen und Zubereitungen der vorliegenden Erfindung, welche Hydroxyflavone der Formel (I) umfassen können auch beispielsweise durch mechanische Mischvorgänge, wobei gleichzeitig auch eine Zerkleinerung der Partikel erfolgen kann, oder mittels Sprühtrocknung hergestellt werden. Bevorzugt sind erfindungsgemäße Zusammensetzungen, die feste Trägerstoffe umfassen und mittels Sprühtrocknung hergestellt sind, die so hergestellten Partikel, besitzen eine mittlere Partikelgröße im Bereich von 30 bis 300 µm und eine Restfeuchte von kleiner oder gleich 5 Gew.-%.

### BEISPIELE

### Beispiel 1: Freisetzung von Serotonin in einem experimentellen Zellsystem mit Homoeriodictyol

Als Zellmodell für die Ausschüttung des Neurotransmitters Serotonin werden humane Neuroblastomazellen (SH-SY5Y, ATCC Nummer CRL-2266) verwendet. Die Kultivierung erfolgt bei 37°C und 5% CO₂-Gehalt mit einer Mischung, die zu gleichen Teilen aus Eagle's Minimum Essential Medium (MEM) und F12 Medium (jeweils mit 10% FBS und 1% Penicillin / Streptomycin) besteht. Für die Messung der Serotoninfreisetzung werden die Zellen mit Trypsin geerntet und nach einer Vitalitätsprüfung durch Trypanblaufärbung in definierter Zellzahl in 35 mm Zellkulturschalen ausgesät.

Nach 5-minütiger Stimulierung von 1,25 * 10⁶ humanen Neuroblastomazellen (SH-SY5Y) mit 300 µL Krebs-Ringer-HEPES Puffer, 0,1% Ascorbinsäure, pH 6,2, mit oder ohne Zusatz des Homoeriodictyols, wobei bei Zusatz des Homoeriodictyols in Konzentrationen von 0,001 µM, 0,01 µM, 0,1 µM, 1 µM und 10 µM im Krebs-Ringer-HEPES Puffer eingestellt wird, wird der Serotoningehalt mit Hilfe eines enzymbasierten Nachweisverfahrens (Serotonin-ELISA sensitiv, DLD Diagnostica, Hamburg, Deutschland) ermittelt. Die Zellen werden mit einem natriumlaurylsarcosinathaltigem Puffer lysiert und der DNA-Gehalt mit Hilfe einer NanoQuant-Platte (Tecan, Mennendorf, Schweiz) zur Normalisierung der Serotoninfreisetzung ermittelt.

**Tabelle 1: Serotonin-Freisetzung SH-SY5Y-Zellen nach Stimulierung mit Homoeriodictyol**

| Testsubstanz | T/C [%] | Standardabweichung [%] |
|---|---|---|
| Kontrolle (mit 0.1% EtOH) | 100 | 14.0 |
| Homoeriodictyol 0,001 µM | 62.2 | 23.4 |
| Homoeriodictyol 0,01 µM | 42.9 | 13.3 |
| Homoeriodictyol 0,1 µM | 42.6 | 11.5 |
| Homoeriodictyol 1 µM | 43.5 | 21.1 |
| Homoeriodictyol 10 µM | 71.9 | 22.3 |

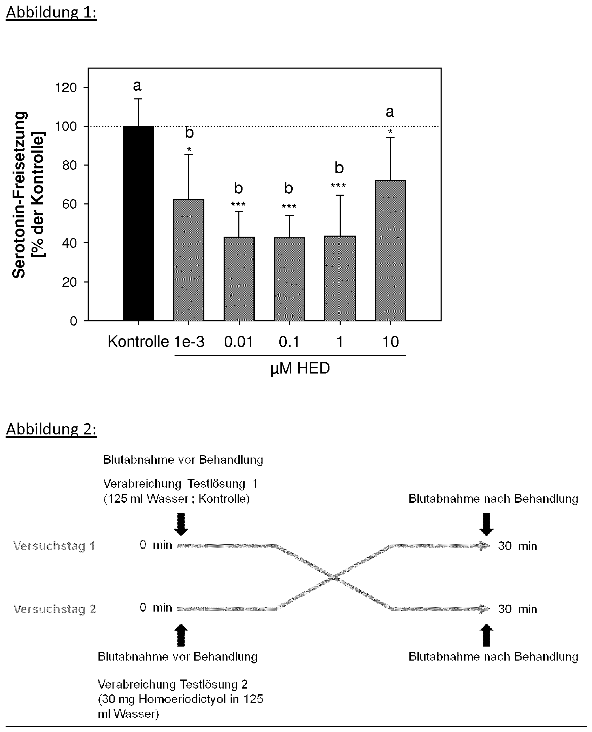

In **Abbildung 1**_wird die_Serotoninausschüttung nach Inkubation mit Homoeriodictyol in den Konzentrationen 0,001 µM, 0,01 µM, 0,01 µM, 1 µM und 10 µM aus neuronalen SH-SY5Y-Zellen dargestellt. Die Ergebnisse sind in % zur Kontrolle (Puffer mit 0,1% Ethanol) dargestellt. n=3, tR=2. Signifikante Unterschiede der Behandlungen zur Kontrolle wurden mit einer einfaktoriellen ANOVA mit anschließendem Holm-Sidak post hoc-Test gegen die Kontrolle getestet und entsprechend folgendem Schema markiert: * p≤ 0,05; ** p.≤ 0,01; ***p≤ 0,001. Konzentrationsabhängige Unterschiede wurden mit einer einfaktoriellen ANOVA mit anschließendem Holm-Sidak post hoc-Test getestet und mit unterschiedlichen Buchstaben (a, b) gekennzeichnet, wobei kein gemeinsamer Buchstabe einen signifikanten Unterschied markiert.

### Beispiel 2: Veränderung der Serotonin-Plasmakonzentration von gesunden Probanden nach Gabe von 125 mL Wasser (Kontrolle) oder 30 mg Homoeriodictyol gelöst in 125 mL Wasser

Zur Ermittlung der Serotonin-Plasmakonzentration wird gesunden Probanden jeweils an einem Versuchstag vor und im Durchschnitt 37 Minuten nach der Verabreichung der Testlösung (1.Versuchstag: 125 mL Wasser; 2.Versuchstag: 30 mg Homoeriodictyol gelöst in 125 mL Wasser) Vollblut abgenommen. Aus dem Vollblut wird durch 15-minütiges, gekühltes Zentrifugieren bei 1800xg das Plasma gewonnen und die Serotonin-Konzentration im Plasma durch ein enzymbasiertes Nachweisverfahren ermittelt (Serotonin-ELISA, DLD Diagnostika, Deutschland).

Die Veränderung der Serotonin-Plasmakonzentration vor und nach Verabreichung von 125 mL Wasser oder 30 mg Homoeriodictyol gelöst in 125 mL Wasser wird in Tabelle 2 wiedergegeben. Es besteht ein signifikanter Unterschied zwischen den beiden Behandlungen (p<0,05; n=10), der durch einen abhängigen, zweiseitigen Student's T-Test ermittelt wurde und mit * markiert ist.

**Tabelle 2: Veränderung der Serotonin-Plasmakonzentration durch Zugabe von Wasser und Homoeriodictyol**

| | 125 mL Wasser | 30 mg Homoeriodictyol in 125 mL Wasser |
|---|---|---|
| Veränderung der Serotonin-Konzentration im Plasma nach Verabreichung der Testlösung | -6,81±10,6 | -19,1±10,6 * |

In **Abbildung 2** ist eine schematische Darstellung des Versuchsablaufs der cross-over Humanintervention zur Bestimmung der Veränderung der Serotonin-Plasmakonzentration 30 min nach Gabe von 125 mL Wasser (Versuchstag 1) oder 30 mg Homoeriodictyol gelöst in 125 mL Wasser (Versuchstag 2).

### Beispiel 3: Verringerung der absoluten Plasma-Serotoninkonzentration nach oraler Gabe von 30 mg Homoeriodictyol

Zur Ermittlung der Serotonin-Plasmakonzentration wird gesunden Probanden an einem Versuchstag vor und im Durchschnitt 35 Minuten nach der Verabreichung von 30 mg Homoeriodictyol gelöst in 125 ml Wasser Vollblut abgenommen. Aus dem Vollblut wird durch 15-minütiges, gekühltes Zentrifugieren bei 1800xg das Plasma gewonnen und die Serotonin-Konzentration im Plasma durch ein enzymbasiertes Nachweisverfahren ermittelt (Serotonin-ELISA, DLD Diagnostika, Deutschland).

Die Veränderung der Serotonin-Plasmakonzentration vor und nach Verabreichung 30 mg Homoerdictyol gelöst in 125 ml Wasser wird in Tabelle 3 wiedergegeben. Es besteht ein signifikanter Unterschied zwischen den beiden Behandlungen (p<0,05, n=16), der durch einen abhängigen, zweiseitigen Student's T-Test ermittelt wurde und mit * markiert ist.

**Tabelle 3: Veränderung der Serotonin-Plasmakonzentration durch Zugabe von Wasser und Homoeriodictyol**

| | Vor | Nach |
|---|---|---|
| | oraler Verabreichung von 30 mg Homoeriodictyol in 125 mL Wasser | oraler Verabreichung von 30 mg Homoeriodictyol in 125 mL Wasser |
| Serotonin-Konzentration im Plasma [nmol] | 306 ± 152 | 192 ± 122 * |

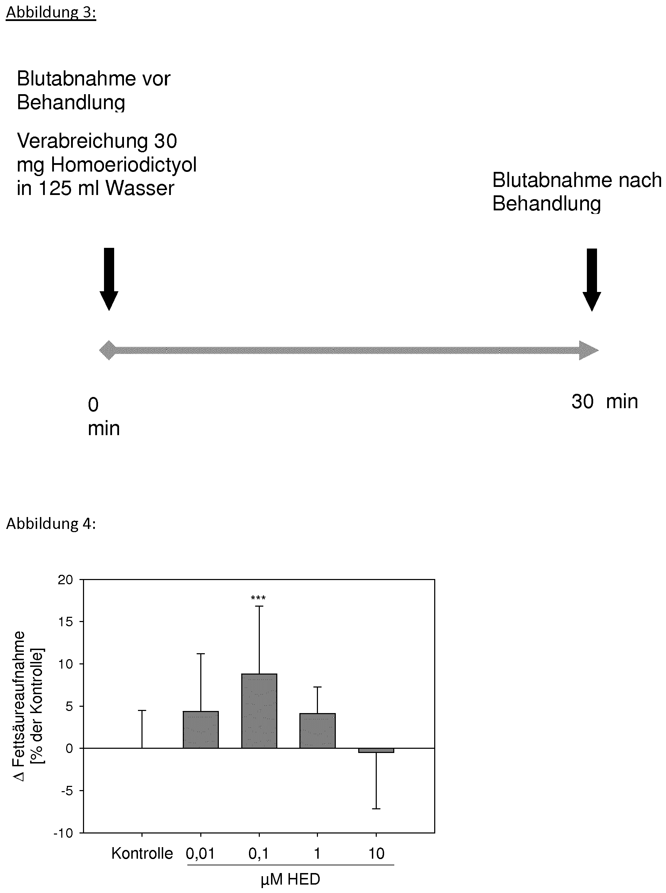

In **Abbildung 3** ist eine schematische Darstellung des Versuchsablaufs zur Bestimmung der absoluten Plasma-Serotonin-Konzentration nach oraler Gabe von 30 mg Homoeriodictyol gelöst in 125 mL Wasser.

### Beispiel 4: Fettsäureaufnahme in einem experimentellen Testsystem mit Homoeriodictyol

Als Zellmodell für die Aufnahme von freien Fettsäure in periphere Zellen, hier Adipozyten, werden 3T3-L1 Preadipozyten (ATCC CL-173) verwendet. Die Kultivierung erfolgt bei 37°C und 5 % CO₂-Gehalt in DMEM-Kulturmedium, das mit 10 % FBS 4% L-Glutamin und 1 % Penicillin / Streptomycin versetzt wurde. Für die Messung der Fettsäureaufnahme werden die 3T3-L1 Zellen in 175 cm² Zellkulturflaschen zu vollständig ausgereiften Adipozyten differenziert. Dazu werden die Zellen zwei Tage nach Erreichen der Konfluenz durch Zusatz von 10 µg/mL Insulin, 1 µM Dexamethason und 0.5 mM Isobutylmethylxanthin zum Kulturmedium für 48 h zur Differenzierung stimuliert. Nachdem die Zellen für weitere 48 h mit 10 µg/mL Insulin im Zellkulturmedium behandelt wurden, wird wieder normales Kulturmedium bis zur vollständigen Ausreifung der Adipozyten eingesetzt. Die Zellen werden üblicherweise sieben bis neun Tage nach Einsetzen der Differenzierung für den Versuch verwendet. Dazu werden die Zellen mit Trypsin geerntet und nach einer positiven Vitalitätsprüfung mit Trypanblau zu je 65,000 Zellen pro Well in einer 96-well Platte ausgestreut.

Die Messung der Fettsäureaufnahme erfolgt nach einer 60-minütigen Synchronisation der Zellen durch Entzug des fötalen Rinderserums und einer 30-minütigen Preinkubation mit Hank's Balanced Salt Solution, versetzt mit 20 mM HEPES (HBSS/HEPES), mit oder ohne Zusatz von 0,01 bis 10 µM Homoeriodyticol mit Hilfe eines fluoreszenzmarkierten Fettsäureanalogs (QBT Fatty Acid Uptake Kit, Molecular Devices, Deutschland). Dargestellt wird die Veränderung der durch den Anstieg des Fluoreszenzsignals entstehende Fläche unter der Kurve über den Verlauf von einer Stunde in % im Vergleich zur Kontrolle.

Die Veränderung der Fettsäureaufnahme in 3T3-L1 Adipozyten nach 30-minütiger Preinkubation mit HBSS/HEPES-Puffer mit 0,1% Ethanol (Kontrolle) oder 0,01-10 µM Homoeriodictyol gelöst in HBSS/HEPES-Puffer wird in Tabelle 4 wiedergegeben. Signifkante Unterschiede (p<0,001) zur Kontrolle wurden mit einer einfaktoriellen ANOVA gegen die Kontrolle mit anschließendem Dunn's post-hok Test getestet und sind mit *** markiert. Dargestellt sind Mittelwerte aus 3-4 unabhänigen Experimenten mit jeweils 3 Wiederholen.

**Tabelle 4: Veränderung der Fettsäureaufnahme durch Zugabe von Homoeriodictyol**

| **Testsubstanz** | **Veränderung in %** | **Standardabweichung [%]** |
|---|---|---|
| EtOH Kontrolle | 0 | 4.48 |
| Homoeriodictyol 0,01 µM | 4.34 | 6.80 |
| Homoeriodictyol 0,1 µM | 8.78*** | 8.05 |
| Homoeriodictyol 1 µM | 4.10 | 3.16 |
| Homoeriodictyol 10 µM | -0.47 | 6.67 |

Die Veränderung der Fettsäureaufnahme in 3T3-L1 Adipozyten nach 30-minütiger Preinkubation mit HBSS/HEPES-Puffer mit 0,1% Ethanol (Kontrolle) oder 0,01-10 µM Homoeriodictyol gelöst in HBSS/HEPES-Puffer wird in **Abbildung 4** wiedergegeben. Signifkante Unterschiede (p<0,001) zur Kontrolle wurden mit einer einfaktoriellen ANOVA gegen die Kontrolle mit anschließendem Dunn's post-hok Test getestet und sind mit *** markiert. Dargestellt sind Mittelwerte aus 3-4 unabhänigen Experimenten mit jeweils 3 Wiederholungen

### Beispiel 5: Plasmakonzentration von Homoeriodictyol nach oraler Gabe von Homoeriodictyol an gesunde Testpersonen

Die Untersuchung der Plasmakonzentration nach oraler Aufnahme von 30 mg Homoeriodictyol, gelöst in 125 mL Wasser, erfolgte durch Abnahme von 4 mL Vollblut in EDTA-Monovetten (Sarstedt, Deutschland) vor und 30 Minuten nach Verabreichung der Homoeriodictyol- Testlösung. Aus dem Vollblut wird durch 15-minütiges, gekühltes Zentrifugieren bei 1800xg das Plasma gewonnen. Durch eine Festphasenextraktion wird das sich im Plasma befindliche Homoerdictyol aufgereinigt und die Konzentration durch LC-MS mit interner Kalibrierung über Eriodictiyol ermittelt.

Die Homoeriodictyol-Plasmakonzentrationen von sieben gesunden Testpersonen 30 min nach oraler Verabreichung von 30 mg HED ist in Tabelle 5 dargestellt. Der Mittelwert wurde aus n=7 gebildet. Der *in vitro* getestete Konzentrationsbereich (Beispiele 1 und 4) entspricht somit der Plasmakonzentration 30 min nach oraler Verabreichung von 30 mg Homoeriodictyol.

**Tabelle 5: Homoeriodictyol-Plasmakonzentrationen von Testpersonen nach oraler Verabreichung von 30 mg HED**

| | **Niedrigste Konzentration [µM]** | **Maximale Konzentration [µM]** | **Mittelwert ± Standardabweichung [µM]** |
|---|---|---|---|
| **Homeriodictyol im Plasma** | 0,01 | 0,08 | 0,04±0,02 |

### Anwendungsbeispiel A-E

### Beispiel A: Erfrischungsgetränke (zuckerhaltig, kalorienreduziert, kalorienfrei)

Die Zutaten wurden in der angegebenen Reihenfolge gemischt und mit Wasser auf 100 % aufgefüllt. Die Mischungen werden in Glasflaschen gefüllt und carbonisiert.

| **Inhaltsstoff** | **Einsatz in Gew.-%** | | | | | | |
|---|---|---|---|---|---|---|---|
| **Zubereitung** | **I** | **II** | **III** | **IV** | **V** | **VI** | **VII** |
| Zucker (Sucrose) | 10 | 10 | 7 | - | - | 8 | 7 |
| Glucose/Fructose-Sirup aus Mais, enthaltend 55 Gew.-% Fructose | - | - | - | - | 10 | - | - |
| Rebaudiosid A 95 % | - | - | 0,02 | 0,05 | - | - | - |
| Zitronensäure | 0,15 | 0,15 | 0,06 | 0,15 | 0,15 | 0,15 | 0,15 |
| Phosphorsäure | - | - | 0,07 | - | - | - | - |
| Zuckercouleur | - | - | 0,14 | - | - | - | - |
| Coffe i n | - | - | 0,01 | - | - | - | - |
| Zitronenaroma | 0,1 | 0,05 | - | 0,1 | 0,1 | 0,1 | 0,1 |
| Limonenaroma | - | 0,05 | - | - | - | - | - |
| Getränke-Emulsion Typ "Cola" | - | - | 0,05 | - | - | - | - |
| Phloretin | - | - | 0,002 | 0,003 | - | 0,002 | 0,001 |
| Hesperetin | - | - | 0,001 | 0,002 | - | - | 0,002 |
| Extrakt aus Rubus suavissimus, enthaltend 5 Gew.-% Rubusosid bez. auf das Gesamtgewicht des Extraktes | - | - | - | - | - | 0,01 | - |
| Homoeriodictyol-Natriumsalz | - | - | 0,010 | 0,005 | - | - | - |
| Wasser | auf 100 auffüllen | | | | | | |

### Beispiel B: Verwendung in einem Kaugummi

Teile A bis D werden gemischt und intensiv geknetet. Die Rohmasse kann z.B. in Form von dünnen Streifen zu verzehrsfertigen Kaugummis verarbeitet werden.

| **Teil** | **Inhaltsstoff** | **Einsatz in Gew.-%** | |
|---|---|---|---|
| | | **VIII** | **IX** |
| A | Kaugummibase, Company "Jagum T" | Auf 100 auffüllen | Auf 100 auffüllen |
| B | Sorbit, pulverisiert | 39,00 | 39,00 |
| | Isomalt® (Palatinit GmbH) | 9,50 | 9,50 |
| | Xylit | 2,00 | 2,00 |
| | Mannit | 3,00 | 3,00 |
| | Aspartam® | 0,10 | 0,10 |
| | Acesulfam® K | 0,10 | 0,10 |
| | Emulgum® (Colloides Naturels, Inc.) | 0,30 | 0,30 |
| C | Sorbitol, 70% | 14,00 | 14,00 |
| | Glycerin | 1,00 | 1,00 |
| D | Pfefferminzaroma | 0,5 | 0,5 |
| | Homoeriodictyol-Natriumsalz | 0,03 | 0,01 |
| | Eriodictyol | 0,0100 | - |

### Beispiel C: Verwendung in Hartkaramellen

Palatinit bzw. der Zucker wurden ggfs. nach Zugabe der Zitronensäure mit Wasser gemischt und die Mischung bei 165 °C aufgeschmolzen und anschließend auf 115°C abgekühlt. Das Aroma und die anderen Bestandteile wurden zugegeben und nach dem Durchmischen in Formen gegossen, nach dem Erstarren aus den Formen entfernt und anschließend einzeln verpackt.

| **Inhaltsstoff** | **Gehalt (Gew.-%)** | | | |
|---|---|---|---|---|
| | **X** | **XI** | **XII** | **XIII** |
| Zucker | 74,50 | - | - | - |
| Palatinit, Typ M | - | 74,00 | 75,50 | 75,00 |
| Zitronensäure | 0,5 | 1,0 | 0,5 | - |
| Farbstoff gelb | - | 0,01 | - | - |
| Farbstoff rot | - | - | 0,01 | - |
| Farbstoff blau | 0,01 | - | - | 0,01 |
| Pfefferminzaroma | 0,1 | - | - | 0,1 |
| Zitronenaroma | - | 0,1 | - | - |
| Rotfruchtaroma | - | - | 0,1 | - |
| Rebaudiosid A 98 % | - | 0,040 | - | 0,040 |
| Balansin A nach [SY317] | - | 0,005 | 0,010 | 0,005 |
| Hesperetin | - | 0,001 | - | 0,001 |
| Phloretin | - | 0,002 | - | - |
| Homoeriodictyol-Natriumsalz | 0,02 | 0,001 | 0,002 | 0,02 |
| Wasser | ad 100 | ad 100 | ad 100 | ad 100 |

### Beispiel D: Fettarme Joghurt

Die Inhaltsstoffe wurden gemischt und auf 5°C gekühlt.

| | **Zubereitung (Gew.-%)** | | | |
|---|---|---|---|---|
| **Inhaltsstoff** | **XIV** | **XV** | **XVI** | **XVII** |
| Sucrose | 10 | 8 | 6 | - |
| Rebaudiosid A 98 % | - | - | - | 0,050 |
| Extrakt aus Rubus suavissimus, enthaltend 5 Gew.-% Rubusosid bezogen auf das Gesamtgewicht des Extraktes, z.B. von PlantExtrakt | - | 0,010 | 0,010 | - |
| Hesperetin | - | 0,001 | 0,001 | 0,002 |
| Phloretin | - | - | 0,002 | 0,002 |
| Homoeriodictyol-Natriumsalz | 0,003 | 0,010 | 0,002 | 0,005 |
| Joghurt, 0,1 % Fett | zu 100 % auffüllen | | | |

### Beispiel E: Fruchtgummi

Polydextrose ist ein nicht süß schmeckendes Polysaccharid mit niedrigem Brennwert.

| | **Zubereitung (Gew.-%)** | |
|---|---|---|
| **Inhaltsstoff** | **XVIII** | **XIX** |
| Saccharose | 34,50 | 8,20 |
| Glucosesirup, DE 40 | 31,89 | 30,09 |
| Iso Syrup C* Tru Sweet 01750 (Cerestar GmbH) | 1,50 | 2,10 |
| Gelatine 240 Bloom | 8,20 | 9,40 |
| Polydextrose (Litesse® Ultra, Danisco Cultor GmbH) | - | 24,40 |
| Gelber und roter Farbstoff | 0,01 | 0,01 |
| Citronensäure | 0,20 | |
| Kirscharoma, enthaltend 1 Gew.-% Homoeriodictyol und 0,3 Gew.-% Hesperetin bezogen auf das Aroma | 0,20 | 0,10 |
| Wasser | ad 100 | ad 100 |

## Patentansprüche

1. Medikament, enthaltend Verbindungen der Formel (I) oder deren Salze, 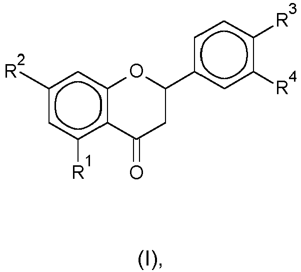 wobei die Verbindungen der Formel (I) ausgewählt sind aus der Gruppe bestehend aus: Homoeriodictyol (1), Eriodictyol (2) und Sterubin (4),
zur Verwendung bei der Behandlung von Appetitlosigkeit.

2. Medikament nach Anspruch 1 zur Verwendung nach Anspruch 1, umfassend
(i) mindestens eine Verbindung nach Anspruch 1 oder deren Salze
(ii) mindestens einen festen Trägerstoff,
(iii) eine oder mehrere Aromastoffe,
mit der Maßgabe, dass sich die Komponenten (i), (ii) und (iii) mit gegebenenfalls weiteren Inhaltstoffen zu 100 Gew.-% addieren, bezogen auf die Gesamtmenge der gesamten Stoffmischung.

3. Medikament nach den Ansprüchen 1 und/oder 2 zur Verwendung nach den Ansprüchen 1 und/oder 2, **dadurch gekennzeichnet, dass** im Salz die Gegenkationen ausgewählt sind aus der Gruppe bestehend aus: Ammoniumionen, Trialkylammoniumionen, Natrium-, Kalium-, Magnesium-, oder Calciumkationen.

4. Nicht-therapeutische Verwendung von Homoeriodictyol (1), Eriodictyol (2), und/oder Sterubin (4) in der Ernährung oder dem Genuss dienenden oral zu konsumierenden Lebensmitteln, zur Beeinflussung des Serotoninspiegels und der Konzentration freier Fettsäuren im Blut zur Steigerung des Appetits.

5. Nicht-therapeutische Verwendung eines der Ernährung oder dem Genuss dienenden Lebensmittels, vorzugsweise eines Nahrungsergänzungsmittels, umfassend
(i) mindestens eine Verbindung der Formel (I) oder deren Salze, 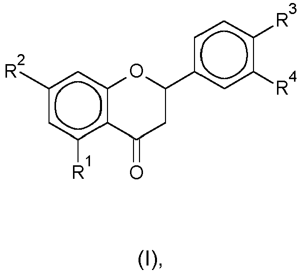 wobei die Verbindungen der Formel (I) ausgewählt sind aus der Gruppe bestehend aus: Homoeriodictyol (1), Eriodictyol (2) und Sterubin (4),
(ii) mindestens einen festen Trägerstoff, und
(iii) einen oder mehrere Aromastoffe,
zur Beeinflussung des Serotoninspiegels und der Konzentration freier Fettsäuren im Blut zur Steigerung des Appetits.

## Claims

1. Medication, comprising compounds of formula (I) or the salts thereof, 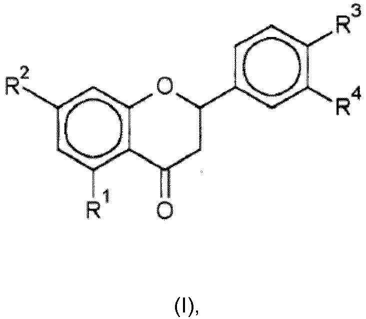 wherein the compounds of formula (I) are selected from the group consisting of: Homoeriodictyol (1), Eriodictyol (2) and Sterubin (4),
for use in the treatment of loss of appetite.

2. Medication according to claim 1 for use according to claim 1, comprising
(i) at least one compound according to claim 1 or the salts thereof
(ii) at least one solid carrier,
(iii) one or more flavouring substances,
providing that the components (i), (ii) and (iii) and where applicable with further ingredients add up to 100 wt.-%, related to the total amount of the total substance mixture.

3. Medication according to claims 1 and/or 2 for use according to claims 1 and/or 2, **characterized in that** the counterions in the salt are selected from the group consisting of ammonium ions, trialkyl ammonium ions, sodium-, potassium-, magnesium- or calcium cations.

4. Non-therapeutic use of Homoeriodictyol (1), Eriodictyol (2) and/or Sterubin (4) in foodstuff serving for nutrition or pleasure and to be consumed orally, for influencing the serotonin level and the concentration of free fatty acids in the blood for increasing the appetite.

5. Non-therapeutic use of a foodstuff serving for nutrition or pleasure, preferably a food supplement, comprising
(i) at least one compound of formula (I) or the salts thereof, 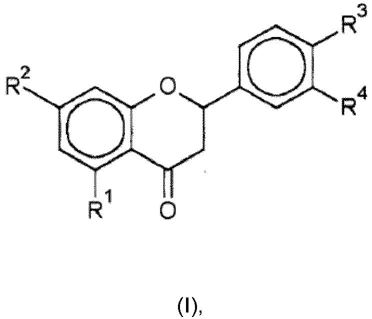 wherein the compounds of formula (I) are selected from the group consisting of: Homoeriodictyol (1), Eriodictyol (2) and Sterubin (4),
(ii) at least one solid carrier, and
(iii) one of more flavouring substances,
for influencing the serotonin level and the concentration of free fatty acids in the blood for increasing the appetite.

## Revendications

1. Médicament contenant des composés de formule (I) ou leurs sels, 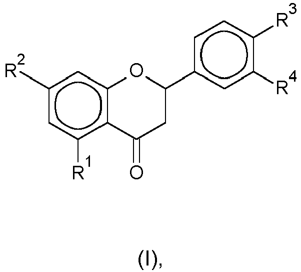 dans lequel les composés de formule (I) sont choisis dans le groupe constitué par: l'homoériodictyol (1), l'ériodictyol (2) et la stérubine (4),
destiné à être utilisé dans le traitement de la perte d'appétit.

2. Médicament selon la revendication 1 pour l'utilisation selon la revendication 1, comprenant
(i) au moins un composé selon la revendication 1 ou ses sels
(ii) au moins un support solide,
(iii) une ou plusieurs substances aromatisantes,
à condition que les composants (i), (ii) et (iii) s'additionnent, avec éventuellement d'autres ingrédients, de manière à donner 100 % en poids, par rapport à la quantité totale du mélange total de substances.

3. Médicament selon les revendications 1 et/ou 2 pour l'utilisation selon les revendications 1 et/ou 2, **caractérisé par le fait que**, dans le sel, les contre-cations sont choisis dans le groupe constitué par: les ions ammonium, les ions trialkylammonium, les cations de sodium, de potassium, de magnésium ou de calcium.

4. Utilisation non thérapeutique d'homoériodictyol (1), d'ériodictyol (2) et/ou de stérubine (4) dans l'alimentation ou des aliments servant au plaisir et à consommer par voie orale, pour influer sur le niveau de sérotonine et sur la concentration d'acides gras libres dans le sang pour augmenter l'appétit.

5. Utilisation non thérapeutique d'un aliment servant à la nutrition ou au plaisir, de préférence d'un complément alimentaire, comprenant
(i) au moins un composé de formule (I) ou ses sels, 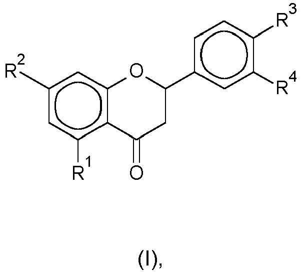 dans laquelle les composés de formule (I) sont choisis dans le groupe constitué par: l'homoériodictyol (1), l'ériodictyol (2) et la stérubine (4),
(ii) au moins un support solide et
(iii) une ou plusieurs substances aromatisantes,
pour influer sur le niveau de sérotonine et sur la concentration d'acides gras libres dans le sang pour augmenter l'appétit.
